# EUROPEAN PATENT SPECIFICATION

(11) **EP 1 267 832 B1**
(45) Date of publication and mention of the grant of the patent: **02.06.2004**
(21) Application number: 01910305.0
(22) Date of filing: 06.03.2001
(51) Int. Cl.: A61K 9/113, A61K 31/04, A61P 29/00

(54) **SELF EMULSIFYING DRUG DELIVERY SYSTEM, wherein the fatty agent is optional**
SELBSTEMULGIERENDES ARZNEISTOFFVERABREICHUNGSSYSTEM, wobei das Fettmittel optional ist
SYSTEME DE DELIVRANCE D'UN MEDICAMENT AUTO-EMULSIFIANT, dans lequel l'agent gras est optionnel

(30) Priority: 08.03.2000 SE 0000773
(43) Date of publication of application: 02.01.2003
(73) Proprietor: AstraZeneca AB, 151 85 Södertälje (SE)
(72) Inventor: HOLMBERG, Christina, S-151 85 Södertälje (SE); SIEKMANN, Britta, S-234 33 Lomma (SE)
(86) International application number: PCT/SE2001/000467
(87) International publication number: WO 2001/066088

(56) References cited:
- WO-A1-95/08983
- WO-A1-95/09831
- WO-A2-99/56727

## Description

### Field of the invention

The present invention is directed to a new pharmaceutical composition in form of an emulsion pre-concentrate, a unit dosage form comprising said composition, its use in therapy as well as a process for the preparation thereof.

### Background and prior art

Non-steroidal anti-inflammatory drugs. commonly abbreviated NSAIDs, are well-known drugs for the treatment of pain and inflammation. One of the major drawbacks with NSAIDs is that they have severe gastro-intestinal side-effects. Patients undergoing treatment with NSAIDs for a longer period of time, such as naproxen, often experience problems with stomach gastrointestinal side-effects.

Nitrogen oxide releasing NSAID compounds (in the following NO-releasing NSAIDs), have recently been found to have an improved side-effect profile, see e.g. WO 94/04484, WO 94/12463, WO 95/09831 and WO 95/30641.

NO-releasing NSAIDs are lipophilic compounds with poor aqueous solubility. They can be classified into class 2 according to the Biopharmaceutical Classification System proposed by Amidon et al. *(Pharm. Res. 12 (1995) pp. 413-420).* Drugs of this class are characterised by low aqueous solubility but reasonably well permeability. A biopharmaceutical problem with these compounds is that their absorption from the gastrointestinal tract (GIT) may be dissolution rate limited, resulting in poor bioavailibility upon oral administration.

WO 95/08983 discloses a self-emulsifying composition for oral administration that forms a microemulsion *in situ* when in contact with biological fluids. This composition can be characterised as a self-microemulsifying drug delivery system (SMEDDS), and comprises at least
- an active compound,
- a lipophilic phase consisting of a mixture of glycerides and fatty acid esters,
- a surface-active agent,
- a cosurfactant, and
- a hydrophilic phase which is achieved after ingestion by the physiological liquid of the digestive medium.

The present invention distinguishes in several aspects from WO 95/08983 and other SMEDDS. Whereas the compositions disclosed in WO 95/08983 form a microemulsion in *situ,* the compositions of the present invention form an emulsion. The SMEDDS of WO 95/08983 require the presence of a lipophilic phase to solubilise the active compound. Such a lipophilic solubiliser phase is not needed for the present invention since the active compound, the NO-releasing NSAID, is able to solely constitute the oil phase of the *in situ* emulsion. Compositions of WO 95/08983 comprise *inter alia* a cosurfactant in addition to a surface-active agent. The presence of a cosurfactant is not necessary for compositions of the present invention reducing toxicological concern to a minimum.

EP 274 870 discloses a pharmaceutical composition comprising a non-steroidal anti-inflammatory drug (NSAID) and a surfactant, the composition being capable of forming micelles containing the NSAID upon oral administration. These micelles have been found to present a particularly appropriate form to administer NSAIDs orally, alleviating their adverse effects on the gastrointestinal tract (GIT). Micelles are aggregates in which the surfactant molecules are generally arranged in a spheroidal structure with the hydrophobic region at the core shielded, in an aqueous solution, from the water by a mantle of outer hydrophilic regions. The drug is usually solubilised in the surfactant. Micelles are to be contrasted in terms of their structure with emulsions which are formed by compositions of the present invention. Whereas micelles are thermodynamically stable one-phase-systems (according to the Gibbs phase law) in which the aggregates usually have a diameter of approximately two lengths of the surfactant molecule forming it, i.e. in the order of some ten to hundred Ångström (Å), emulsions are much larger aggregates, in the order of nanometers to micrometers in diameter, consisting of an oily core which is surrounded by one or several layers of surfactants. Emulsions are generally two-phase-systems, and they are thermodynamically unstable (but may be kinetically stable). Another major difference between the compositions of EP 274 870 and the present invention is the nature of the active compound. Whereas NSAIDs are crystalline powders by nature, the NO-releasing NSAIDs or mixtures of NO-releasing NSAIDs used in the present invention are in oil form or a thermosoftening semisolid. Moreover, micelles usually require a much higher drug surfactant ratio compared to the oil surfactant ratio required to form an emulsion.

One of the unique features with NO-releasing NSAIDs is that many of these compounds are oils or thermosoftening semisolids which are practically insoluble in water. With high-dose NO-releasing NSAIDs. e.g. when the dose is above about 350 mg, it is difficult to formulate a tablet of reasonable size of the large amount of oii or semisolid. The lipophilic NO-releasing NSAIDs can, however, be formulated as oil-in-water emulsions where the compound constitutes, or is part of, the oil phase emulsified in water by one or more surfactants.

WO 99/56727 describes an emulsion preconcentrate comprising a poorly watersoluble therapeutic agent, an oil component having an HLB ≤ 4 and a surfactant having an HLB between 10 and 20. It is mentioned that these hydrophilic solvents having an HLB > 20 destabilize the emulsion. The hydrophilic solvents mentioned are ethanol, propylene glycol, polyethylene glycol and polyoxypropylene block polymers.

It is further stated that these hydrophilic solvents should be used, if at all, in an amount of less then 10% by weight in order to prevent the emulsion from destabilizing.

In pharmacokinetic animal studies it has been surprisingly found that such oil-in-water emulsions of NO-releasing NSAIDs display a much better bioavailability compared to the unemulsified substance. A problem with emulsions is, however, that they are thermodynamically unstable and have a poor long-term storage stability since they often tend to coalescence, creaming/sedimentation or phase separation. Moreover, they do not represent a convenient dosage form for oral administration since often large volumes are needed to incorporate one dose, and unpleasant bitter or soapy taste may be a major problem. It is *inter alia* not possible to fill oil-in-water emulsions into gelatine capsules since the high water content of the emulsion is incompatible with the capsule shell and would dissolve it.

### Outline of the invention

The problems mentioned above have now been solved by providing a novel Self Emulsifying Drug Delivery System, commonly known as SEDDS, suitable for oral administration. More particularly, the present invention is directed to a pharmaceutical composition suitable for oral administration, in form of an emulsion pre-concentrate, comprising
(i) one or more NO-releasing NSAID(s);
(ii) one or more surfactants:
(iii) optionally an oil or semi-solid fat;
said composition forming an *in-situ* oil-in-water emulsion upon contact with aqueous media such as gastrointestinal fluids.

The composition according to the present invention may optionally further comprise one or more short-chain alcohols.

The composition will form an *in situ* oil-in-water emulsion of small droplets of nanometer to micron size upon contact with gastrointestinal fluids, the droplets being constituted of one or more NO-releasing NSAIDs forming the core of the droplet which is covered by one or several layers of surfactant. The *in situ* formed oil-in-water emulsion will provide a good bioavailability of the NO-releasing NSAID upon oral administration. Storage stability of the emulsion is not a concern since the emulsion is not formed until the pre-concentrate has been taken by the patient, i.e. first at the moment of administration. The possibly unpleasant taste of the pre-concentrate is not a problem when filled into capsules.

The pharmaceutical composition according to the present invention is an emulsion pre-concentrate at the time of administration to a patient. The emulsion pre-concentrate can be filled into single unit dosage forms such as capsules, drinking ampoules and dose cushions. or may alternatively be formed as other suitable dosage forms such as chewable soft pills and chewy-base lozenges.

Upon contact with aqueous media such as gastrointestinal fluids, the emulsion pre-concentrate transforms into an oil-in-water emulsion. Thus, the composition will form an *in-situ* oil-in-water emulsion in the gastrointestinal tract (GI tract). The drug release rate of the composition is determined by the droplet size of the *in situ* emulsion and the polarity of the emulsion droplets, the latter being governed by the hydrophilic-lipophilic balance (HLB) of the drug/surfactant mixture, and the concentration of the surfactant. Generally, small droplet size and high polarity gives rise to a high drug release rate *(N.H. Shah et al.*, *Int. J. Pharm. 106 (1994), pp. 15-23)*

The wording "NSAID" is defined as a non-steroidal anti-inflammatory drug, i.e. any drug having an anti-inflammatory effect, but which compound does not belong to the compound class "steroids". A person skilled in the art will know whether a compound falls under the definition NSAID. Examples of specific NSAIDs are naproxen, diclofenac, aceclofenac, indomethacine, ketorolac, sulindac, meloxicam, piroxicam, tenoxicam, ibuprofen, ketoprofen, naproxen, azapropazon, nabumeton, carprofen, tiaprofenic acid, suprofen, indoprofen, etodolac, fenoprofen, fenbufen, flurbiprofen, bermoprofen, pirazolac, zaltoprofen, nabumetone, bromfenac, ampiroxicam, and lornoxicam. This list should however not be considered as exhaustive in any way. The wording "NO-releasing NSAID" is contemplated to include any non-steroidal anti-inflammatory drug (NSAID), a salt or an enantiomer thereof, which has the capability to release nitrogen oxide.

NO-releasing NSAIDs are lipophilic compounds with poor aqueous solubility. They can be classified into class 2 according to the Biopharmaceutical Classification System proposed *by Amidon et al. (Pharm. Res. 12 (1995) 413-420).* Drugs of this class are characterised by low aqueous solubility but reasonably well permeability. A biopharmaceutical problem with these compounds is that their absorption from the gastro-intestinal tract (GIT) may be dissolution rate limited resulting in poor bioavailibility upon oral administration.

Preferred NO-releasing NSAIDs in accordance with the present invention, are compounds of the formula I 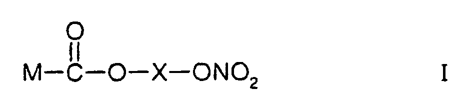 wherein
X is a spacer, i.e. a compound forming a bridge between the nitrogen oxide donating group and the NSAID: and
M is selected from anyone of

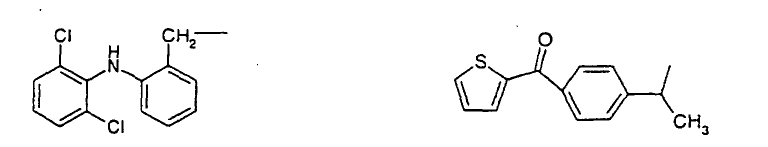

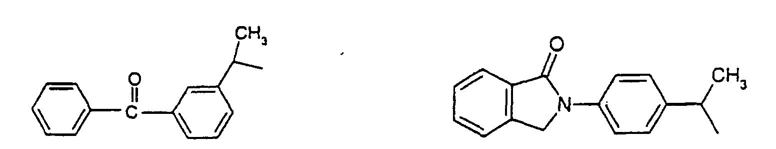

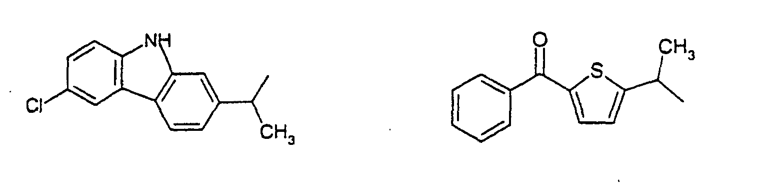

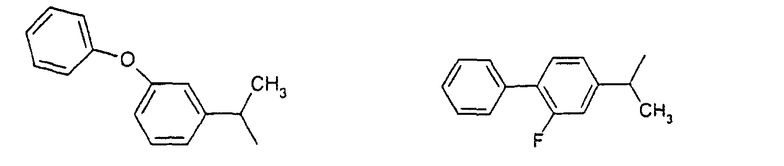

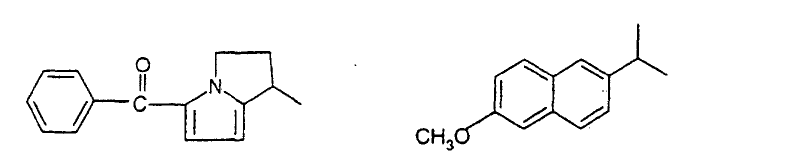

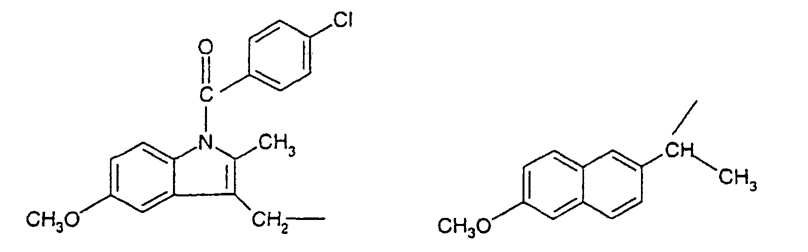

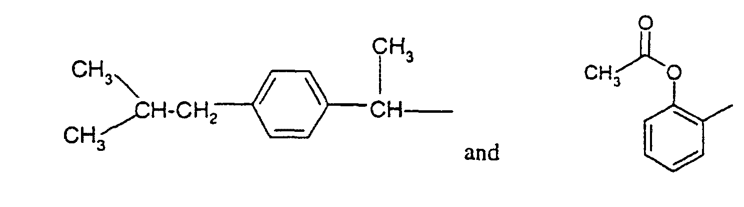

In a preferred embodiment of the invention, the spacer X is selected from a linear, branched or cyclic alkylene group -(CH₂)-ₙ wherein n is an integer of from 2 to 10; and -(CH₂)m-O-(CH₂)p- wherein m and p are integers of from 2 to 10; and -CH₂-pC₆H₄-CH₂-.

In one embodiment of the invention, NO releasing NSAIDs contemplated as active compound(s) in the SEDDS formulation according to the present invention, are compounds disclosed and claimed in WO 94/04484, WO 94/12463, WO 95/09831 and WO 95/30641, which are hereby incorporated by reference.

Specific NO-releasing substances useful in accordance with the present invention are

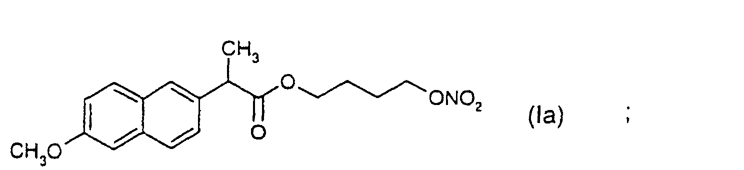

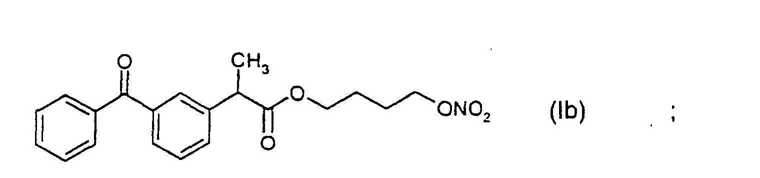

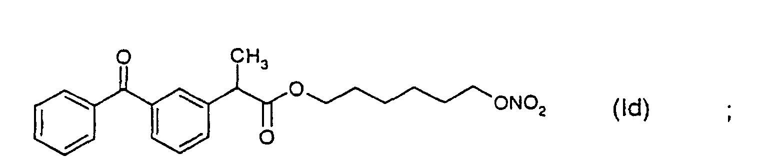

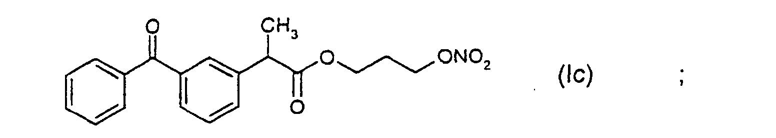

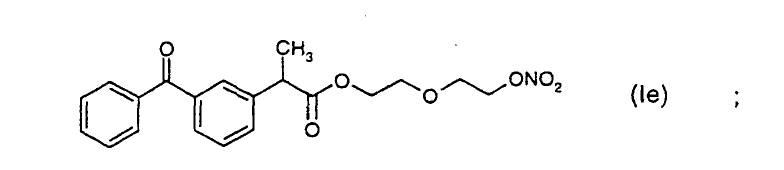

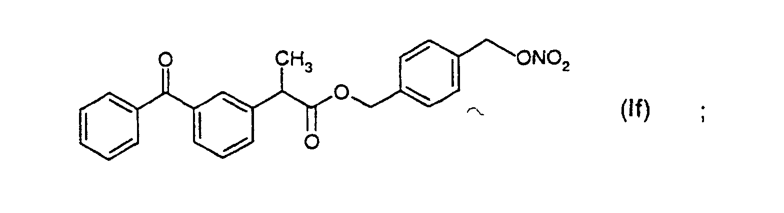

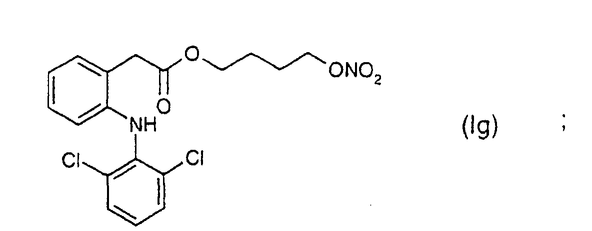

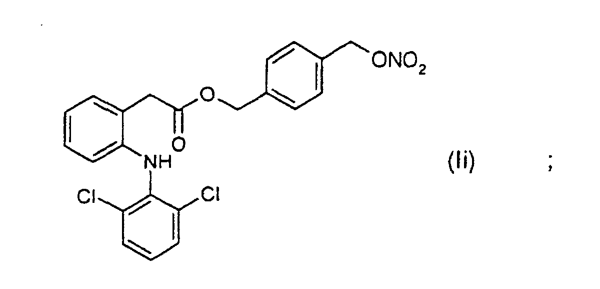

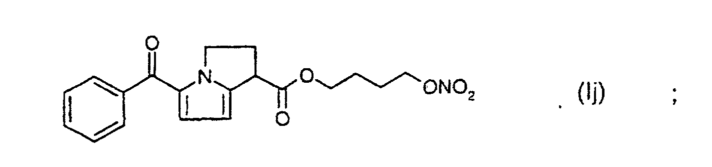

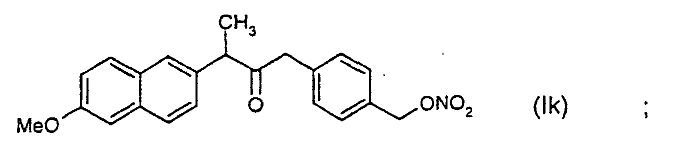

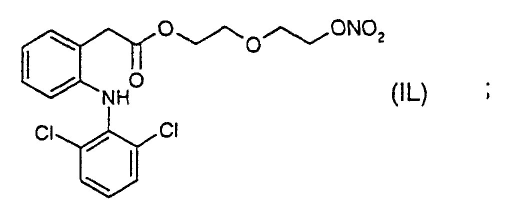

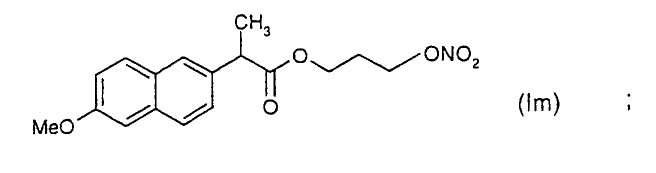

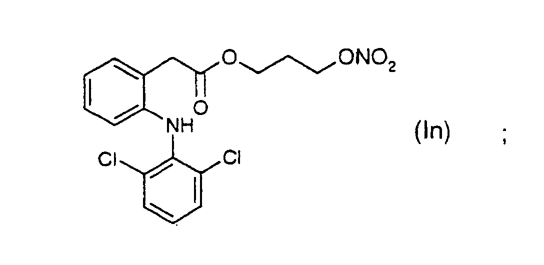

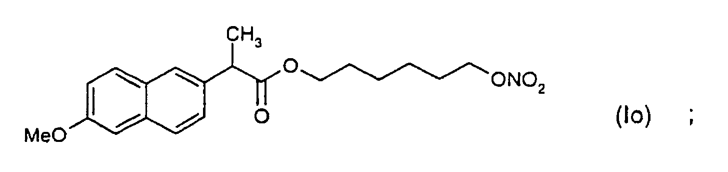

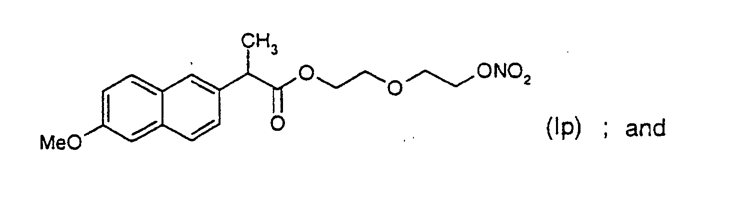

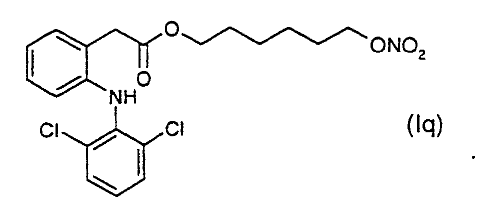

NSAIDs are by nature in form of a powder, whereas NO-releasing NSAIDs predominantly provide a compound in semi-solid or oil form as such, due to the spacer. This unique feature provides the advantage that no external lipophilic oil or semisolid matrix needs to be added to the emulsion pre-concentrate, since this is an inherent feature of the drug. Additionally, a pharmacologically inert oil or semisolid fat may be added to the pharmaceutical composition by means of a filler or as a viscosity regulator. A filling agent may be required to increase dosing accuracy for low dose compounds. A viscosity regulator may be required in order to adjust optimal viscosity for filling of the composition into e.g. capsules. In particular high speed liquid filling of capsules requires careful adjustment of viscosity within a range that prevents splashing on the low viscosity end and thread-formation on the high viscosity end. Moreover, the viscosity range must be chosen so as to give a pumpable formulation. The viscosity range typically required for liquid filling of capsules is from 0. 1 to 25 Pa s.

The total amount of NO-releasing NSAID(s) used in the composition of the invention is preferably in the range 50-1500 mg per unit dose. In still a further preferred embodiment, the amount of NO-releasing NSAID(s) used in the composition is 125-500 mg per unit dose.

The wording "unit dose" is defined as the amount of active compound administered in one single capsule, or dissolved in one glass of water.

The wording "surfactant" is defined as surface-active amphiphilic compounds such as block co-polymers. Preferred surfactants in accordance with the present invention are non-ionic surfactants, for example those containing polyethylene glycol (PEG) chains, particularly block co-polymers such as poloxamers.

Examples of suitable poloxamers are Poloxamer 407 (Pluronic F127®): Poloxamer 401 (Pluronic L121®): Poloxamer 237 (Pluronic F87®); Poloxamer 338 (Pluronic F138® ): Poloxamer 331 (Pluronic L101®): Poloxamer 231 (pluronic L81®); tetrafunctional polyoxyethylene polyoxypropylene block copolymer of ethylene diamine, known as Poloxamine 908 (Tetronic 908®); Poloxamine 1307 (Tetronic 1307®); Poloxamine 1107 polyoxyethylene polyoxybutylene block copolymer, known as Polyglycol BM45®. This list is only intended to serve as exemplification of surfactants that may be used in accordance with the present invention, and should not in any way be considered as exhaustive or as limiting the invention.

All surfactants described above are commercially available from e.g. BASF, Dow Chemicals, and Gattefossé.

The total amount of surfactant(s) in accordance with the invention may be within the range of from 12.5-6000 mg, preferably of from 100-500 mg.

The ratio NO-releasing NSAID:surfactant may vary from 1:0.1 to 1:10, preferably from 1:0.3 to 1:3.

If an additional oil is added to the pharmaceutical composition this may be any oil as long as it is inert and compatible with the capsule material, as well as being acceptable for use in pharmaceuticals. A person skilled in the art will appreciate which oil to select for the intended purpose. Examples of suitable oils that may be used in accordance with the present invention are vegetable oils such as coconut oil. corn oil, soybean oil, rape seed oil, safflower oil and castor oil. Also animalic oils such as fish oil and triglycerides are suitable for the purposes of the present invention.

If a semi-solid fat is used as a filler for the pharmaceutical composition, this may preferably be selected from mono-. di- and triglycerides, and fatty acid alcohol such as stearyl alcohol. Gelucires 33/01®, 39/01®, 43/01® , glyceryl palmitostearate such as Precirol ATO5®. Gelucire is a mixture obtained by mixing mono-, di-. and tri-esters of glycerol, mono- and di-esters of PEG. or free PEG.

In one aspect of the present invention, an oily (lipophilic) or semi-solid NO-releasing NSAID is used as the active ingredient.

If an additional oil or semi-solid fat is used in the pharmaceutical composition according to the invention, this may serve as a filler or as a viscosity regulator.

The wording "short-chain alcohols" used in accordance with the present invention is herein defined as linear or branched mono-, di- or tri-alcohols having 1-6 carbon atoms. Examples of such short-chain alcohols useful in accordance with the invention are ethanol, propylene glycol and glycerol.

If a short-chain alcohol is added to the pharmaceutical composition according to the invention, the solubility is enhanced and a smaller amount of surfactant is required.

In another aspect of the invention, two or more NO-releasing NSAIDs are used as active ingredients, where anyone of said drugs may be present as an oil or as a semi-solid, or where at least one of said drugs is present as an oil or as a semi-solid and the other one(s) may be present as a solid which is dissolved or suspended in the oily or semi-solid compound. Combinations of two or more NO-releasing NSAIDs may be advantageous in case the high NO-load of a high-dose low potent NO-releasing NSAID is desired to be supplemented with a low dose of high potent NO-releasing NSAID.

A further aspect of the invention is a combination of one or more NO-releasing NSAIDs and an acid susceptible proton pump inhibitor (PPI) compound. The NO-NSAIDs should be formulated such that it is emulsified in the stomach, i.e. as a SEDDS formulation as described above, while the acid susceptible proton pump inhibitor (PPI) must be protected from contact with the acidic gastric juice by for instance an enteric coating. The enteric coating layered PPI remain unaffected until it reaches the intestine, where the PPI is released. Individually prepared enteric coating layered units of the proton pump inhibitor (PPI) may be mixed into the SEDDS melt. Alternatively the PPI's may be filled into a capsule filled with solidified SEDDS, where a layer of protective paraffin may be needed between SEDDS and the prepared PPI pellets. In still an alternative embodiment the prepared PPI pellets may be mixed into a liquid SEDDS formulation.

The combination may thus either be a fix combination, i.e. as a formulation where the NO-releasing NSAID(s) and the acid susceptible proton pump inhibitor are mixed and thereafter filled into a suitable dosage unit. In an alternative embodiment of the invention the acid susceptible proton pump inhibitor may be filled into a capsule with an already solidified SEDDS formulation of one or more NO-releasing NSAID(s) - in this case a layer of protective paraffin or other inert material may be required between the SEDDS formulation and the acid susceptible proton pump inhibitor. In still an alternative embodiment the acid susceptible proton pump inhibitor is mixed into a liquid SEDDS formulation of the NO-releasing NSAID(s).

In an alternative embodiment of the invention, the NO-releasing NSAID(s) and the PPI may be provided in form of a kit, where the NO-releasing NSAID and the PPI are administered sequentially, i.e. one after the other. The order of administration is not crucial, meaning that either of the NO-releasing NSAID or the PPI may be administered before the other. Thus, one embodiment of the invention comprises a combination treatment where one or more NO-releasing NSAIDs are administered to a patient in need of treatment, whereafter a PPI is administered, or vice versa.

Examples of proton pump inhibitors suitable in a combination with a NO-releasing NSAID in accordance with the present invention as stated above, is a compound of the general formula I or a pharmaceutically acceptable alkaline salt thereof, or one of its single enantiomer or an alkaline salt of the single enantiomer: 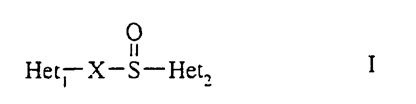 wherein
Het₁ is 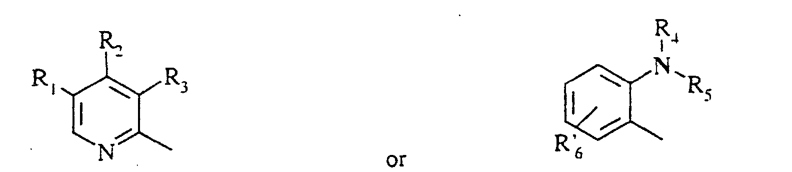
Het₂ is 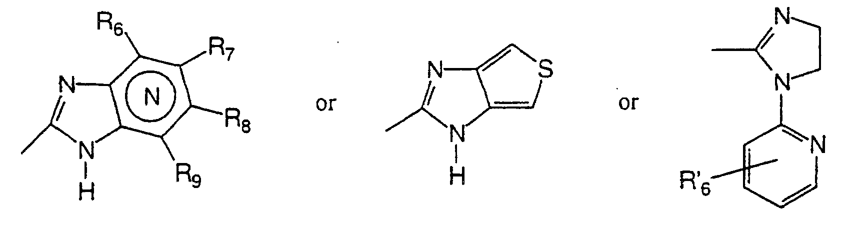
X= 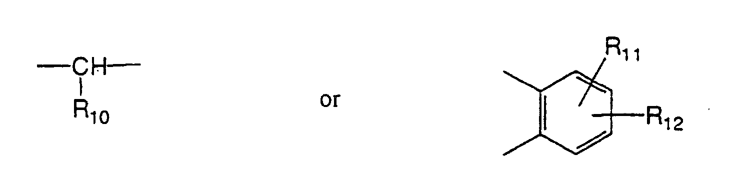
wherein
N in the benzimidazole moiety means that one of the carbon atoms substituted by R₆-R₉ optionally may be exchanged for a nitrogen atom without any substituents;
R₁, R₂ and R₃ are the same or different and selected from hydrogen, alkyl, alkoxy optionally substituted by fluorine, alkylthio, alkoxyalkoxy, dialkylamino, piperidino, morpholino, halogen, phenyl and phenylalkoxy;
R₄ and R₅ are the same or different and selected from hydrogen, alkyl and aralkyl;
R₆' is hydrogen, halogen, trifluoromethyl, alkyl and alkoxy;
R₆-R₉ are the same or different and selected from hydrogen, alkyl, alkoxy, halogen, haloalkoxy, alkylcarbonyl, alkoxycarbonyl, oxazolyl, trifluoroalkyl, or adjacent groups R₆-R₉ form ring structures which may be further substituted;
R₁₀ is hydrogen or forms an alkylene chain together with R₃ and
R₁₁ and R₁₂ are the same or different and selected from hydrogen, halogen or alkyl; alkyl groups, alkoxy groups and moities thereof, they may be branched or straight C₁- C₉ -chains or comprise cyclic alkyl groups, such as cycloalkyl-alkyl.

Examples of specific proton pump inhibitors suitable in accordance with the present invention are

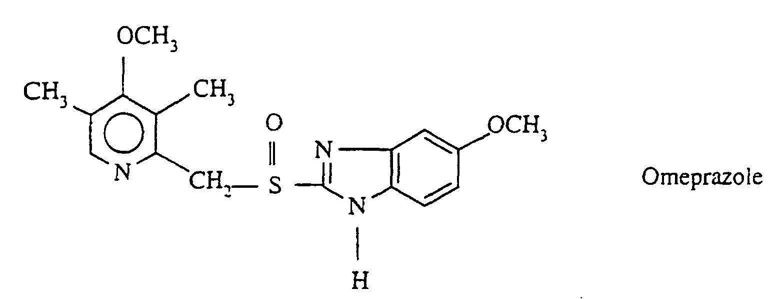

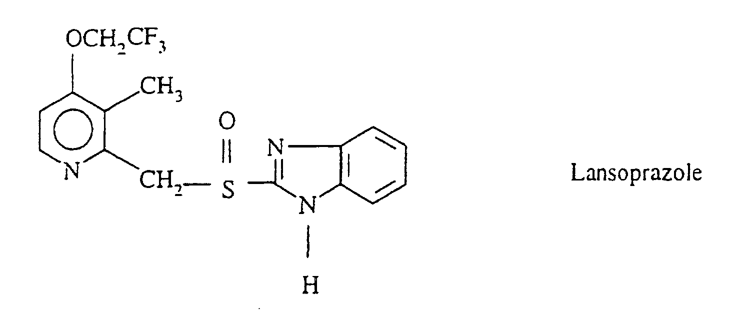

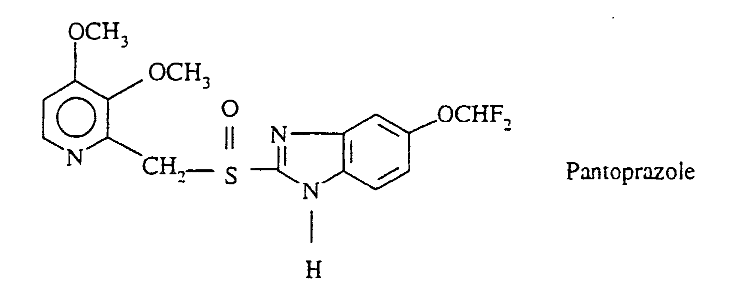

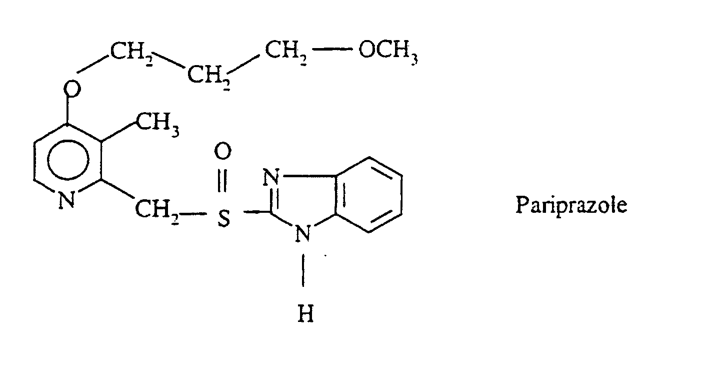

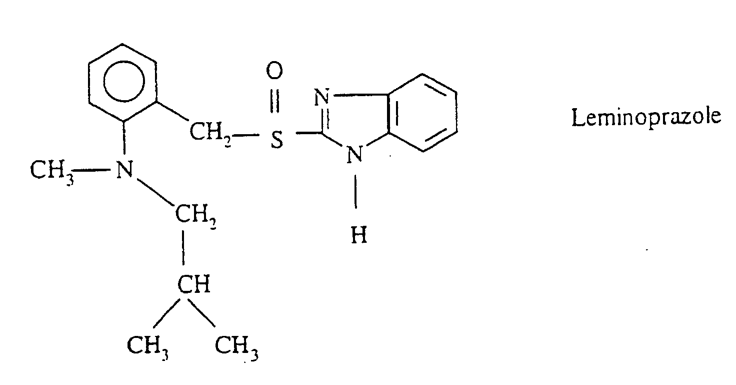

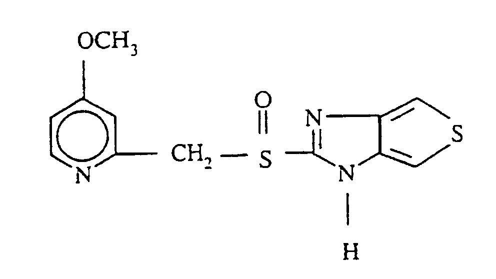

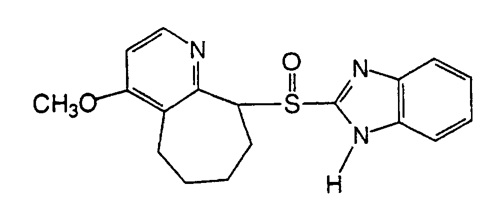

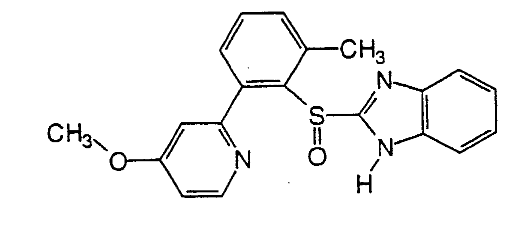

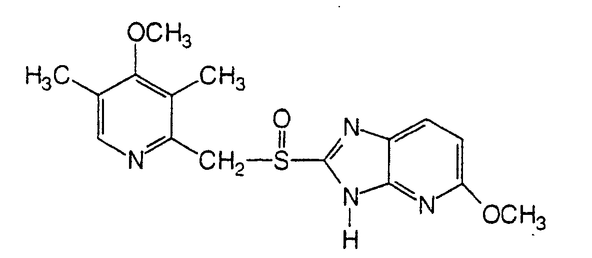

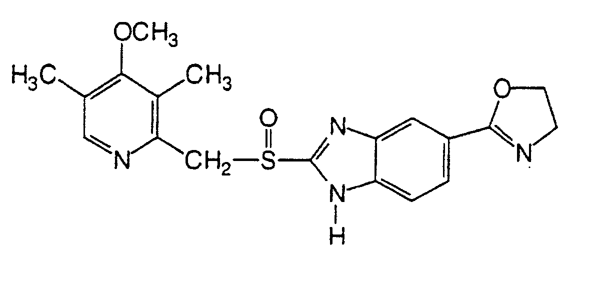

The acid susceptible proton pump inhibitors used in the dosage forms of the invention may be used in their neutral form or in the form of an alkaline salt, such as for instance the Mor²⁺, Ca²⁺, Na⁺, K⁺ or Li⁺ salts, preferably the Mg²⁺ salts. Further where applicable, the compounds listed above may be used in racemic form or in the form of the substantially pure enantiomer thereof, or alkaline salts of the single enantiomers.

Suitable proton pump inhibitors are for example disclosed in EP-A1-0005129, EP-A1-174 726, EP-A1-166 287, GB 2 163 747 and WO 90/06925, and further especially suitable compounds are described in WO 95/01977 and WO94/27988.

The proton pump inhibitors used in a combination in accordance with the present invention, are preferably provided as enteric coating layered pellets comprising the acid susceptible proton pump inhibitor. For the composition of the enteric coating layered pellets and its preparation, reference is made to WO 96/01623, which is hereby incorporated by reference.

Suitable combinations in accordance with the present invention are for instance a NO-releasing NSAID of the formula Ia and omeprazole or an alkaline salt of omeprazole, (S)-omeprazole or an alkaline salt of (S)-omeprazole; or a NO-releasing NSAID of the formula Ii and omeprazole or an alkaline salt of omeprazole, (S)-omeprazole or an alkaline salt of (S)-omeprazole.

The pharmaceutical composition of the invention is filled into single dosage forms suitable for oral administration, such as capsules, drinking ampoules and dose cushions, or may be formulated as other suitable oral dosage forms such as chewable soft pills and chewy-base lozenges.

In a preferred embodiment of the invention, the pharmaceutical composition is filled into hard gelatin capsules, but capsules from alternative materials such as methylcellulose-based shells, and soft gelatine capsules may also be used.

In an alternative embodiment of the invention, the pharmaceutical composition may be dissolved in e.g. a glass of water, thus allowing the pre-concentrate to form an emulsion which may be administered as such. The compositions intended for dissolution prior to administration may be filled e.g. into soft gelatine capsules, plastic or aluminium cushions, or plastic or glass ampoules. This feature is particularly advantageous for high dose compositions which would require a large capsule, for patients who have difficulty in swallowing capsules, and for pediatric patients.

In a preferred embodiment the pharmaceutical composition of the present invention is filled into capsules. Preferred capsules are gelatin capsules which may be soft or hard. The hard gelatine capsule consists of two pieces, a cap and a body, one fitting inside the other. The hard gelatine capsules are produced empty and filled in a separate operation step. The soft gelatin capsule is a capsule which is manufactured and filled in one single operation.

As mentioned above, the emulsion pre-concentrate transforms into an oil-in-water emulsion upon contact with the gastrointestinal fluids, whereby the active drug is released.

Thus, the composition will form an *in situ* oil-in-water emulsion in the gastrointestinal tract (Gl tract).

The pharmaceutical composition of the present invention is particularly useful in the treatment of pain and inflammation. The wording "pain" is intended to include nociceptive and neuropathic pain or combinations thereof: acute, intermittent and chronic pain; cancer pain; migraine and headaches of similar origin. The wording "inflammation" is intended to include rheumatoid arthritis: ostheoarthritis; and juvenile arthritis.

### Methods of preparation

The pharmaceutical composition of the present invention may be prepared mainly by the following alternative methods:

### I. Mixing

a) The oily or semi-solid NO-releasing NSAID is put in a vessel, solid or semi-solid surfactant and solid/oily fat (optional) is added. The mixture is heated to the temperature corresponding to the melting point of the excipients, making the formulation fluid, mixed thoroughly until homogenous (visual inspection) and the pre-concentrate is filled into capsules suitable for oral administration.
b) Alternatively, the oily NO-releasing NSAID is put in a vessel and fluid surfactant is added. The mixture is mixed thoroughly until homogenous (visual inspection) and the pre-concentrate is filled into capsules suitable for oral administration.
c) In a further alternative method. the oily NO-releasing NSAID is put in a vessel, finely grinded (particle size < 177 um) solid surfactant is added. The liquid mixture is mixed thoroughly until homogenous (visual inspection) and the pre-concentrate is filled into capsules suitable for oral administration.
d) In still an alternative method the semi-solid/solid surfactant (s) is put in a vessel. and one or more alcohols are added. The mixture is heated to the temperature corresponding to the melting point of the excipients, making the formulation fluid, mixed thoroughly until homogenous (visual inspection). The NO-NSAID is added, and the mixture is mixed thoroughly until homogenous (visual inspection). The pre-concentrate is filled into capsules suitable for oral administration.
e) In yet a further alternative method the liquid surfactant(s) is put in a vessel, and one or more alcohols are added. The mixture is blended thoroughly until homogenous (visual inspection). The NO-NSAID is added, and the mixture is mixed thoroughly until homogenous (visual inspection). The pre-concentrate is filled into capsules suitable for oral administration.

In order to fill a two-piece capsule or a softgel capsule with a liquid, the formulation must be within a certain viscosity range, as determined by the manufacturer, at the filling temperature suitable for the process. For a two-piece capsule the maximum filling temperature is roughly 70 °C. The viscosity of the formulation should normally be in the range 50-1000 cPoise (=0.05-1 Pas) at the temperature chosen for the filling process.
For the filling of the formulation into softgel capsules, process temperature is not allowed to exceed 30-40 °C (the exact temperature depending on the manufacturer). The formulation must be liquid and have a viscosity that allows it to be pumpable at the filling temperature. In order to make the formulation liquid with an acceptable viscosity, several additives may be used. for example Cremophor EL®.

### II. Filling

For the filling procedure it is required that the composition is in liquid form at the temperature of filling. Semisolid thermosoftening compositions are therefore filled above the liqueifying temperature. Soft gelatine capsules are manufactured and filled in one operation, and may be filled at temperatures of up to 40°C, whereas hard gelatine capsules may be filled at temperatures of up to 70 °C. Hard gelatin capsules filled with compositions that remain liquid at storage temperature require sealing, e.g. by gelatin banding, to prevent leakage. The process of liquid filling of hard gelatin capsules and product requirements are e.g. described in *W.J. Bowtle. Pharmaceutical Technology Europe. October 1998: V.M. Young. Pharmaceutical Manufacturing and Packaging Sourcer, March 1999;* and *E. T. Coole, Pharmaceutical Technology International, September*/*October 1989.* Using two piece capsules permits filling of more than one phase into a single capsule, which may be desired for bi-or multiphase drug release *(W.J. Bowtle et al..Int. J. Pharm. 141 (1996). pp. 9-16).* Several phases of solidifying material can be filled in single steps. The final phase may be liquid if required. The number of phases is only restricted by the capsule size, and volume of the single phases. This special feature may also allow controlled release or separation of different drug substances formulated in the same capsule. Additionally, capsules may be processed further, e.g. by enteric coating.

### III. Combination with PPI' s

The oily or semi-solid NO-releasing NSAID is put in a vessel. solid or semi-solid surfactant and solid/oily fat (optional) is added. The mixture is heated to the temperature corresponding to the melting point of the excipients, making the formulation fluid, mixed thoroughly until homogenous (visual inspection) and prepared enteric coating layered pellets comprising an acid susceptible proton pump inhibitor are added to the mixture. The pre-concentrate with the suspended PPI-pellets is filled into capsules, where it solidifies, suitable for oral administration.

Alternatively the oily or semi-solid NO-releasing NSAID is put in a vessel, solid surfactant and solid/oily fat (optional) is added. The mixture is heated to the temperature corresponding to the melting point of the excipients, making the formulation fluid, mixed thoroughly until homogenous (visual inspection). The pre-concentrate is filled into capsules suitable for oral administration, where it solidifies. A protective layer of paraffin, or any other inert thermosoftening base suitable for oral administration, is added and allowed to solidify. On top of the paraffin, the prepared PPI-pellets are added.

In still an alternative method, the oily NO-releasing NSAID is put in a vessel and fluid surfactant is added. The mixture is mixed thoroughly until homogenous (visual inspection), and the prepared PPI-pellets are added to the mixture. The pre-concentrate with suspended PPI-pellets is filled into capsules suitable for oral administration.

### IV. Characterisation of the formulations

In order to characterise formulations, the time required for the formulation to form an oil-in-water emulsion upon contact with simulated gastric fluid. SGF, (without enzymes), is determined, and the formed emulsion is characterised. SGF comprises of 7 millilitres concentrated hydrochloric acid, 2 grams of sodium chloride and distilled water to give the solution a total volume of 1 L. The "emulsion forming test" is performed in test tubes (beaker) with magnetic stirring. The test tube, containing a small magnet, is filled with 12.5 ml SGF without enzymes, corresponding to one tenth of the average volume of gastric fluid in humans, and formulation corresponding to one tenth of the dose of active compound is added. If the formulation being characterised is a combination with a PPI, the PPI-pellets are checked in order that they are unaffacted by the SGF, which is made by visual inspection. If the enteric coating of the PPI-pellets is affected, the PPI may be affected negatively in pH=1.2, and this can be observed as a marked change in colour.

The time for emulsion formation will vary from 30 seconds and up to 15 minutes, depending on the composition of the formulation. If one or more short-chain alcohols are added, the time for emulsion formation will vary between 2-3 seconds and 3-4 minutes. Also the average particle size of the formed emulsion is studied with Laser Diffraction, LD, or Photon Correlation Spectroscopy, PCS. Depending on particle size either of the two methods may be used.

### Detailed description of the invention

The invention will now be described in more detail by the following examples, which are not to be construed as limiting the invention.

The following emulsion pre-concentrates were prepared.

In the Examples 1-7 below, the active compound used in the formulations was a compound of the formula (Ia) above.

### Example 1

| | | amount [g] |
|---|---|---|
| (i) | Compound of formula (Ia) | 1000 |
| (ii) | Pluronic F127® | 1000 |

A semi-solid formulation was obtained by melting 1 kg of Pluronic F127® (Poloxamer 407) by heating to 62°C. The melt was stirred thoroughly to ensure that no solid particles were present.
1 kg of the compound of formula (Ia) was added to the melted Pluronic F127®, and the mixture was allowed to reach a temperature of 62°C. The liquid formulation was mixed until homogenous (checked by visual inspection). The resulting liquid formulation was then filled into hard gelatin capsules. The formulation becomes a semi-solid upon cooling (in the capsule).

### Characterization

150 milligram of the formulation was put in 12.5 millilitres of SGF (without enzymes) and magnetic stirring. The following results were obtained:

| | |
|---|---|
| Time to emulsion | 13 minutes |
| Average particle size | 2-3 µm |

The viscosity was measured in a Stress Tech cone and plate viscometer, measurement system C 40 4 PC, at the shear rate 20 s⁻¹. The flow was more or less Newtonian.

### Example 2

| | | amount [g] |
|---|---|---|
| (i) | Compound of formula (Ia) | 1000 |
| (ii) | Pluronic L121® | 1000 |

A liquid formulation was prepared by mixing 1 kg of the liquid surfactant Poloxamer 401, with 1 kg of the compound of formula (Ia) at room temperature. The liquid formulation was mixed until homogenous (checked by visual inspection). The resulting liquid formulation was then filled into hard gelatin capsules.

### Characterization

150 milligram of the formulation was put in 12.5 millilitres of SGF (without enzymes) and magnetic stirring. The following results were obtained:

| | |
|---|---|
| Time to emulsion | 20 seconds |
| Average particle size | 11 µm |

### Example 3

| | | amount [g] |
|---|---|---|
| (i) | Compound of formula (Ia) | 1000 |
| (ii) | Polyglycol BM 45 ® | 1000 |
| (iii) | Sodium dodecyl sulphate | 40 |

A formulation was obtained by mixing 1 kg of Polyglycol BM 45® (Poloxamine 1107). 40 grams of sodium dodecyl sulphate, acting as a co-surfactant, and 1 kg of the compound of formula (Ia). The liquid formulation was mixed until homogenous (checked by visual inspection). The resulting liquid formulation was then filled into hard gelatin capsules.

### Characterization

150 milligram of the formulation was put in 12.5 millilitres of SGF (without enzymes) and magnetic stirring. The following results were obtained:

| | |
|---|---|
| Time to emulsion | 15 minutes |
| Average particle size | 0.7 µm |

### Example 4

| | | amount [g] |
|---|---|---|
| (i) | Compound of formula (Ia) | 1000 |
| (ii) | Pluronic F127® | 500 |
| (iii) | Cremophor EL® | 500 |

To be able to fill the semi-solid formulation into soft gelatin capsules, process temperatures must be below 30-40 °C ( the specific temperature depends on manufacturer). This means that the formulation must be fluid and pumpable below 30-40 °C. To obtain a formulation fluid at this temperature, some of the surfactant was replaced with Cremophor EL®. A melt was prepared as described in Example 1, except for the substitution of 0.5 kg surfactant with the same amount of Cremophor EL®.

### Characterization

150 milligram of the formulation was put in 12.5 millilitres of SGF (without enzymes) and magnetic stirring. The following results were obtained:

| | |
|---|---|
| Time to emulsion | 9 minutes |
| Average particle size | 4-5 µm |

### Example 5

| | | amount [g] |
|---|---|---|
| (i) | Compound of formula (Ia) | 1250 |
| (ii) | Pluronic F127® | 1500 |
| (iii) | Fractionated coconut oil | 1880 |

To ensure that low dose formulations will have a good filling precision, and to fill a capsule of a certain volume to minimise the amount of air present, the active compound may be filled up to volume with aliquot part coconut oil. A semi-solid formulation was obtained by melting 1500 kg of Pluronic F127® (Poloxamer 407) by heating to 62°C. The melt was stirred thoroughly to ensure that no solid particles were present. 1.250 kg of the compounf of formula (Ia) and 1880 kg of fractionated coconut oil were added to the melted Pluronic F127® , and the mixture was allowed to reach a temperature of 62°C. The liquid formulation was mixed until homogenous (checked by visual inspection). The resulting liquid formulation was then filled into hard gelatin capsules.

### Characterization

One tenth of the formulation was put in 12.5 millilitres of SGF (without enzymes) and magnetic stirring. The following results were obtained:

| | |
|---|---|
| Time to form emulsion | 10 minutes |
| Average particle size | 5 µm |

### Example 6

| | | amount [g] |
|---|---|---|
| (i) | Compound of formula (Ia) | 62.5 |
| (ii) | Pluronic F127® | 375 |
| (iii) | Fractionated coconut oil | 312.5 |

The formulation was prepared as described for Example 5 above.

### Characterization

Characterization was performed as for Example 5 above. The following results were obtained:

| | |
|---|---|
| Time to form emulsion | 10 minutes |
| Average particle size | 36 µm |

### Example 7

| | | amount [g] |
|---|---|---|
| (i) | Compound of formula (Ia) | 62.5 |
| (ii) | Pluronic F127® | 375 |
| (iii) | Fractionated castor oil | 312.5 |

The formulation was prepared as described for Examples 5 above.

### Characterization

Characterization was performed as for Example 5 above. The following results were obtained:

| | |
|---|---|
| Time to form emulsion | 10 minutes |
| Average particle size | 81 µm |

### Example 8

The active compound of formula (Ib) above was used in the formulation of the present Example 8.

| | | amount [g] |
|---|---|---|
| (i) | Compound of formula (Ib) | 75 |
| (ii) | Polyglycol BM45® | 75 |

A formulation was prepared in the same way as for the preceding Examples.

### Characterization

| | |
|---|---|
| Time to form emulsion | 1.5 minutes |
| Average particle size | 5 µm |

### Example 9

The active compound of formula (Ic) above was used in the formulation of the present Example 9.

| | | amount [g] |
|---|---|---|
| (i) | Compound of formula (Ic) | 75 |
| (ii) | Polyglycol BM45® | 75 |

A formulation was prepared in the same way as for the preceding Examples.

### Characterization

| | |
|---|---|
| Time to form emulsion | 3 minutes |
| Average particle size | 2 µm |

### Example 10

The active compound of formula (Id) above was used in the formulation of the present Example 10.

| | | amount [g] |
|---|---|---|
| (i) | Compound of formula (Id) | 75 |
| (ii) | Polyglycol BM45® | 75 |

A formulation was prepared in the same way as for the preceding Examples.

### Characterization

| | |
|---|---|
| Time to form emulsion | 0.5 minutes |
| Average particle size | 2 µm |

### Example 11

The active compound of formula (Ie) above was used in the formulation of the present Example 11.

| | | amount [g] |
|---|---|---|
| (i) | Compound of formula (Ie) | 75 |
| (ii) | Polyglycol BM45® | 75 |

A formulation was prepared in the same way as for the preceding Examples.

### Characterization

| | |
|---|---|
| Time to form emulsion | 1 minute |
| Average particle size | 4 µm |

### Example 12

The active compound of formula (If) above was used in the formulation of the present Example 12.

| | | amount [g] |
|---|---|---|
| (i) | Compound of formula (If) | 75 |
| (ii) | Polyglycol BM45® | 75 |

A formulation was prepared in the same way as for the preceding Examples.

### Characterization

| | |
|---|---|
| Time to form emulsion | 1 minute |
| Average particle size | 2 µm |

### Example 13

The active compound of formula (Ig) above was used in the formulation of the present Example 13.

| | | amount [g] |
|---|---|---|
| (i) | Compound of formula (Ig) | 75 |
| (ii) | Polyglycol BM45® | 75 |

A formulation was prepared in the same way as for the preceding Examples.

### Characterization

| | |
|---|---|
| Time to form emulsion | 3 minutes |
| Average particle size | 1 µm |

### Example 14

The active compounds of formulas (Ia) and (Ik) above were used in the formulation of the present Example 14.

| | | amount [g] |
|---|---|---|
| (i) | Compound of formula (Ia) | 250 |
| (ii) | Compound of formula (Ik) | 8 |
| (iii) | Pluronic L121® | 250 |

A formulation was prepared by dissolving the compound of formula (Ih) in the compound of formula (Ia), whereafter the Pluronic L121® (Poloxamer 401) was added to this mixture. The liquid formulation was mixed until homogenous (checked by visual inspection).

### Characterization

The formulation was put in 20 ml of SGF (without enzymes) under magentic stirring. The time to emulsion formation was determined. The following results were obtained:

| | |
|---|---|
| Time to form emulsion | 5-10 seconds |

### Example 15

The active compounds of formulas (Ia) and (Ii) above were used in the formulation of the present Example 15

| | | amount [g] |
|---|---|---|
| (i) | Compound of formula (Ia) | 250 |
| (ii) | Compound of formula (Ii) | 8 |
| (iii) | Pluronic L121® | 250 |

A formulation was prepared as described for Example 14.

### Characterization

Performed as in the previous Example 14 above.

| | |
|---|---|
| Time to form emulsion | 3 minutes |

### Example 16

| | | amount [g] |
|---|---|---|
| (i) | Compound of formula (Ia) | 750 |
| (ii) | Pluronic F127® | 450 |
| (iii) | Omeprazole | 20 |

A semi-solid formulation was obtained by melting 450 g Pluronic F127® (Poloxamer 407) by heating to 62 °C. The melt was stirred thoroughly to ensure that no solid particles were present. 750 g of a compound of formula (Ia) above were added to the melted Pluronic F127®, and the mixture was allowed to reach a temperature of 62°C. 20 g Omeprazole in the form of prepared enteric coating layered pellets comprising omeprazole Mg salt, prepared as described in WO 96/01623, Example 2, was added. The liquid formulation was mixed until homogenous (checked visual inspection) and filled into hard gelatine capsules. The formulation became a semi-solid upon cooling (in the capsule).

### Characterization

120 mg of formulation was put in 12.5 ml of SGF (without enzymes) at 37 °C, and magnetic stirring. The SEDDS formed an emulsion upon contact with SGF, and the PPI-pellets remained unaffected by the SEDDS and the pH=1.2, as seen by no change of colour. The time for emulsion formation was 12 minutes.

### Example 17

| | | amount [g] |
|---|---|---|
| (i) | Compound of formula (Ia) | 750 |
| (ii) | Pluronic L121® | 450 |
| (iii) | Omeprazole | 20 |

A liquid formulation was prepared by mixing 450 g of the liquid surfactant Poloxamer 401, with 750 g of a compound of the formula (Ia) above, at room temperature. 20 g Omeprazole in the form of enteric coating layered pellets comprising omeprazole Mg salt, prepared as described in WO 96/01623, Example 2, was added to the mixture. The liquid formulation was mixed until homogenous (checked by visual inspection) and filled into hard gelatine capsules.

### Characterization

120 mg formulation was put in 12.5 ml of SGF (without enzymes) at 37 °C, and magnetic stirring. The SEDDS formed an emulsion upon contact with SGF, and the PPI-pellets remained unaffected by the SEDDS and the pH=1.2. as seen by no change of colour. The time for emulsion formation was 0.5 minutes.

### Example 18

| | | amount [g] |
|---|---|---|
| (i) | Compound of formula (Ia) | 3 |
| (ii) | Pluronic L127® | 0.843 |
| (iii) | sorbitanmonolaurat | 0.282 |
| (iv) | glycerol | 0.375 |

A semi-solid formulation was obtained by melting 0.843 gram of Pluronic F127® (Poloxamer 407), 0.282 gram of sorbitanmonolaurat and 0.375 gram of glycerol by heating to 62°C. The melt was stirred thoroughly to ensure that no solid particles were present. 3 Grams of the compound of formula (Ia) was added to the mixture. The mixture was allowed to reach a temperature of 62°C. The liquid formulation was mixed until homogenous (checked by visual inspection). The resulting liquid formulation was allowed to cool to a temperature of 30 °C. and was then filled into soft gelatin capsules. The formulation becomes a semi-solid upon cooling (in the capsule).

### Characterization

112 milligram of the formulation was put in 12.5 millilitres of SGF (without enzymes) and magnetic stirring. The following result was obtained:

| | |
|---|---|
| Time to emulsion | 2.5-3.5 minutes |

### Example 19

| | | amount [g] |
|---|---|---|
| (i) | Compound of formula (Ia) | 3 |
| (ii) | Pluronic L127® | 0.843 |
| (iii) | sorbitanmonolaurat | 0.282 |
| (iv) | propylene glycol | 0.375 |

A semi-solid formulation was obtained by melting 0.843 gram of Pluronic F127® (Poloxamer 407), 0.282 gram of sorbitanmonolaurat and 0.375 gram of propylene glycol by heating to 62°C. The melt was stirred thoroughly to ensure that no solid particles were present. 3 Grams of the compound of formula (Ia) was added to the mixture. The mixture was allowed to reach a temperature of 62°C. The liquid formulation was mixed until homogenous (checked by visual inspection). The resulting liquid formulation was allowed to cool to a temperature of 30°C, and was then filled into soft gelatin capsules. The formulation stays liquid upon cooling (in the capsule).

### Characterization

112 milligram of the formulation was put in 12.5 millilitres of SGF (without enzymes) and magnetic stirring. The following result was obtained:

| | |
|---|---|
| Time to emulsion | within 20 seconds |

### Example 20

| | | amount [g] |
|---|---|---|
| (i) | Compound of formula (Ia) | 3 |
| (ii) | Pluronic L101® | 0.506 |
| (iii) | sorbitanmonolaurat | 0.169 |
| (iv) | ethanol | 0.225 |

A liquid formulation was prepared. A solution of 0.506 gram of Pluronic L101® (Poloxamer 331), 0.169 gram of sorbitanmonolaurat and 0.225 gram of ethanol, was mixed until homogenous (checked by visual inspection). 3 Grams of the compound of formula (Ia) was added to the mixture, at room temperature. The resulting liquid formulation was then filled into soft gelatin capsules.

### Characterization

97 milligram of the formulation was put in 12.5 millilitres of SGF (without enzymes) and magnetic stirring. The following result was obtained:

| | |
|---|---|
| Time to emulsion | within 20 seconds |

### In vivo study of formulations in mini pigs

A bioavailability study of formulations according to the present invention was performed after oral administration in fastened minipigs.

6 male Göttingen SPF minipigs were used in the study. At the start of the acclimatization period, the animals were 4 months old and had a weight of from 7.7 to 10.1. kg.
The animals were fasted for 12 hours before treatment and until the blood sample at 4 hours post treatment had been taken. A supply of autoclaved hay was given daily as well. Twice daily, the animals were offered domestic quality drinking water.

A pharmaceutical composition of the invention, filled in a suitable unit dosage form according to the invention, was administered to each animal. The dose levels were approximately 15 µmol/kg body weight. 10 ml of tap water was given to facilitate the swallowing of the capsule or corresponding unit dosage.

All visible signs of ill health and any behavioural changes were recorded daily. Any deviation from normal was recorded with respect to time of onset, duration and severity. Included in the daily health check were observations of the consistency of faeces. All animals were weighed on arrival and of the first day of of each treatment.

Blood samples (5 ml) were taken from the jugular vein into Vacutainer tubes containing heparin. Blood samples were taken before treatment (0) and at 15, 30 and 45 minutes; 1, 1.5, 2, 4, 7 and 24 hours after treatment.

## Claims

1. A pharmaceutical composition suitable for oral administration, in form of an emulsion pre-concentrate, comprising
(i) one or more NO-releasing NSAID(s), wherein the NO-releasing NSAID is a compound of the formula I 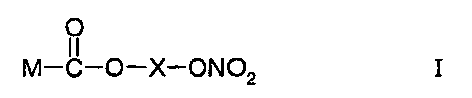 wherein
X is a spacer; and
M is selected from anyone of 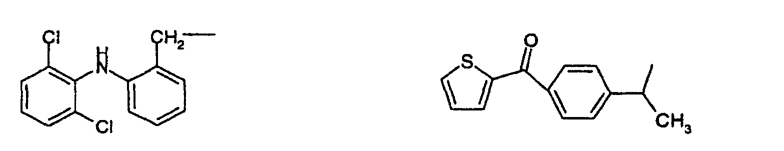 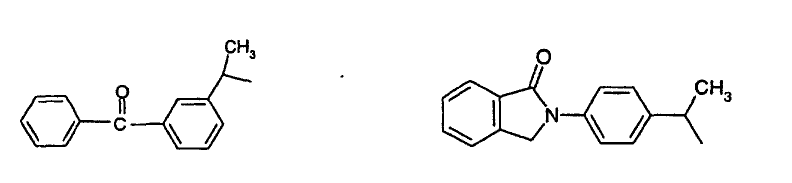 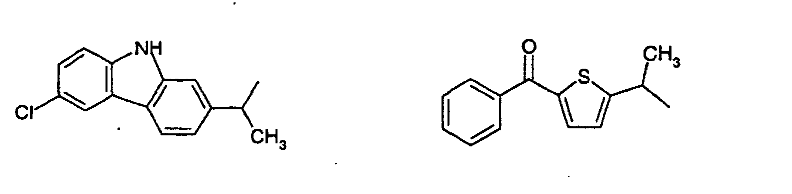 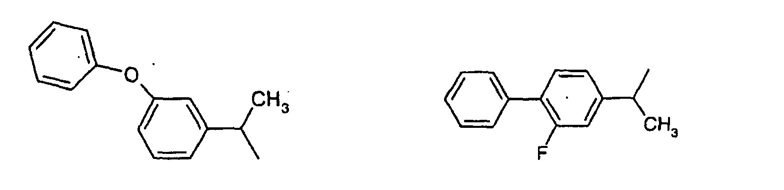 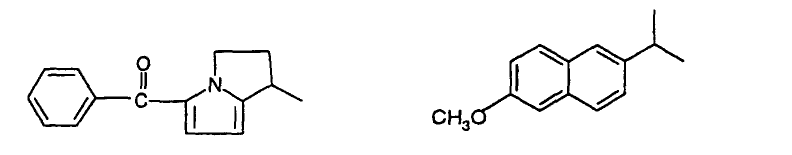 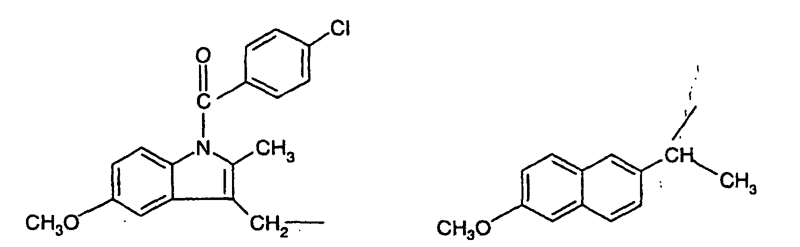 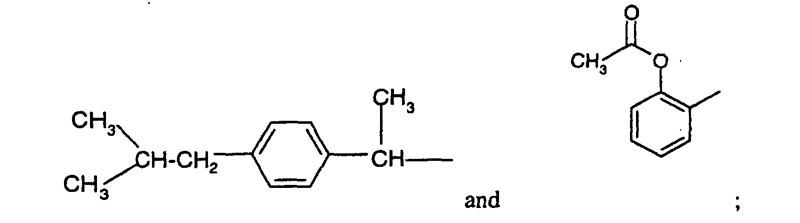
(ii) one or more surfactants;
(iii) optionally an oil or semi-solid fat;
said composition forming an *in-situ* oil-in-water emulsion upon contact with aqueous media such as gastrointestinal fluids.

2. A pharmaceutical composition according to claim 1, further comprising one or more short-chain alcohols.

3. A pharmaceutical composition according to claim 1 or 2, wherein the spacer X of the NO-releasing NSAID is selected from a linear, branched or cyclic alkylene group -(CH₂)-ₙwherein n is an integer of from 2 to 10; -(CH₂)ₘ-O-(CH₂)ₚ- wherein m and p are integers of from 2 to 10; and -CH₂-pC₆H₄-CH₂-.

4. A pharmaceutical composition according to any one of the preceding claims, wherein the NO-releasing NSAID is any one compound selected from 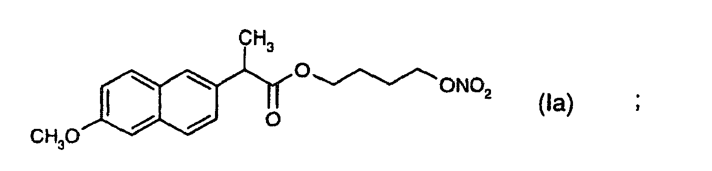 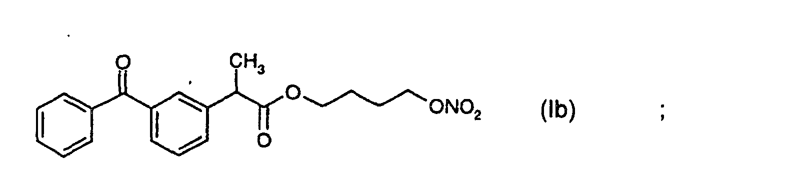 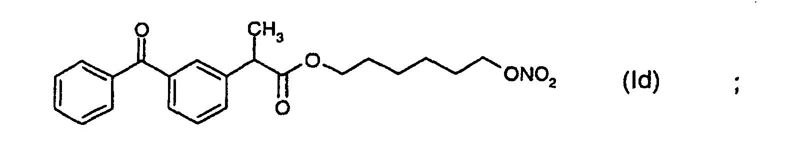 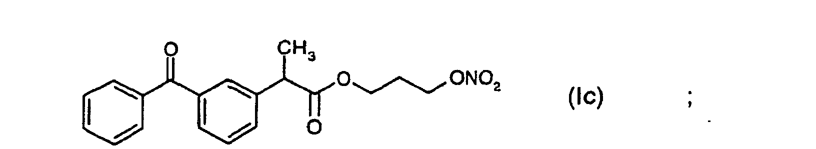 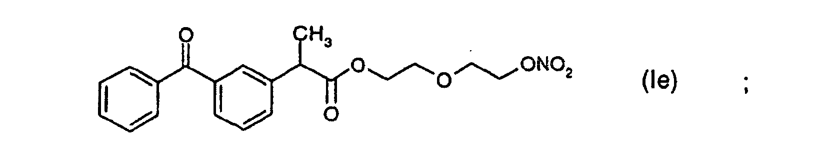 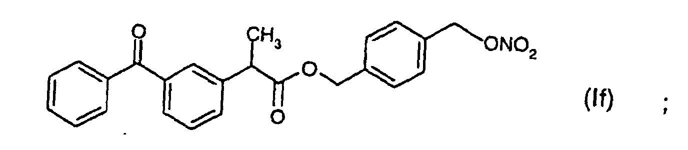 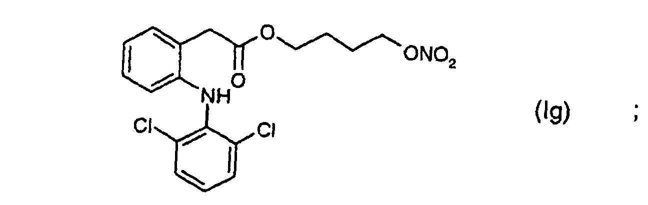 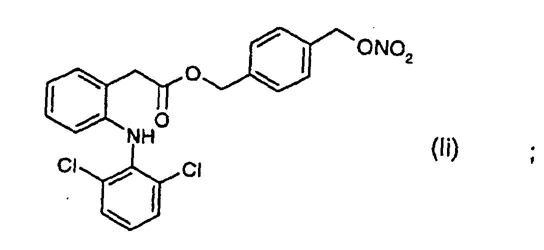 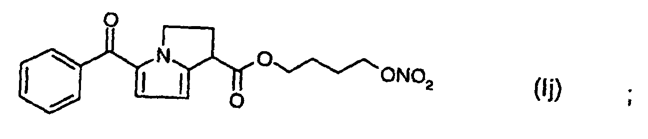 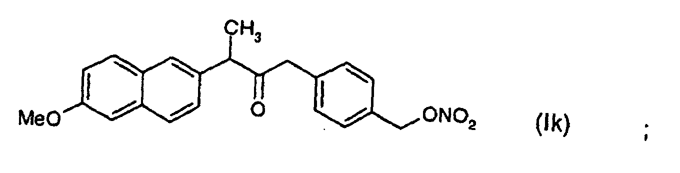 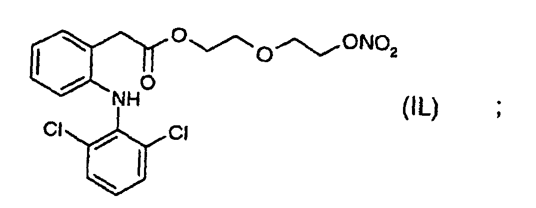 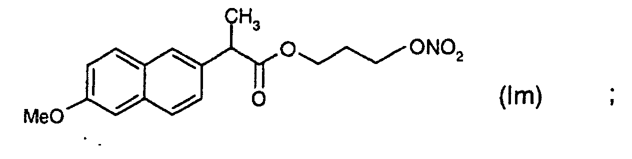 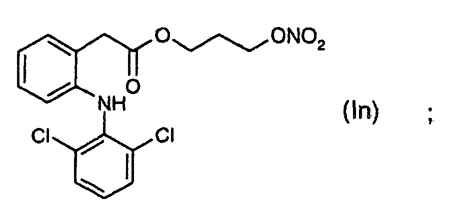 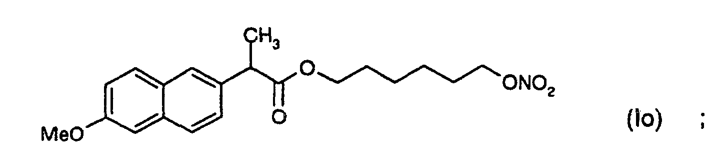 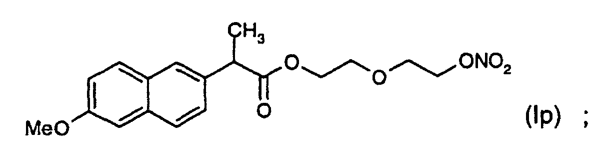 and

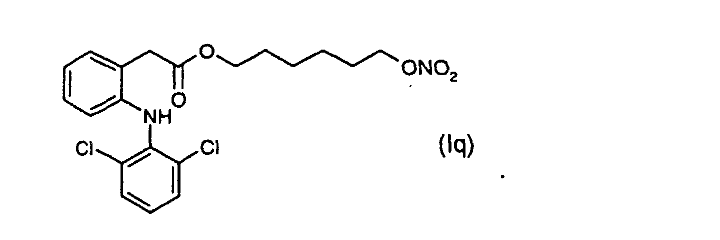

5. A pharmaceutical composition according to any one of the preceding claims, further comprising individually enteric coating layered units of an acid susceptible proton pump inhibitor, or a pharmaceutically acceptable alkaline salt thereof.

6. A pharmaceutical composition according to claim 5, wherein the acid susceptible proton pump inhibitor is selected from a compound of the general formula I or a pharmaceutically acceptable alkaline salt thereof, or one of its single enantiomer or an alkaline salt of the single enantiomer 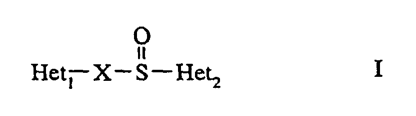 wherein
Het₁ is 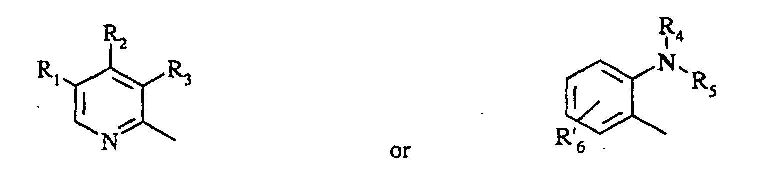
Het₂ is 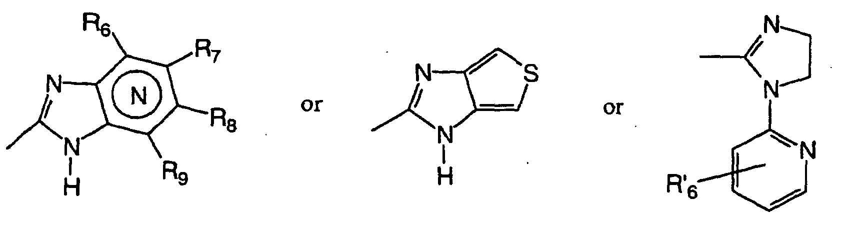
X= 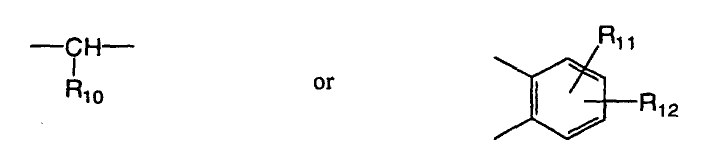
wherein
N in the benzimidazole moiety means that one of the carbon atoms substituted by R₆-R₉ optionally may be exchanged for a nitrogen atom without any substituents;
R₁, R₂ and R₃ are the same or different and selected from hydrogen, alkyl, alkoxy optionally substituted by fluorine, alkylthio, alkoxyalkoxy, dialkylamino, piperidino, morpholino, halogen, phenyl and phenylalkoxy;
R₄ and R₅ are the same or different and selected from hydrogen, alkyl and aralkyl;
R₆' is hydrogen, halogen, trifluoromethyl, alkyl and alkoxy;
R₆-R₉ are the same or different and selected from hydrogen, alkyl, alkoxy, halogen, haloalkoxy, alkylcarbonyl, alkoxycarbonyl, oxazolyl, trifluoroalkyl, or adjacent groups R₆-R₉ form ring structures which may be further substituted;
R₁₀ is hydrogen or forms an alkylene chain together with R₃ and
R₁₁ and R₁₂ are the same or different and selected from hydrogen, halogen or alkyl; alkyl groups, alkoxy groups and moities thereof , they may be branched or straight C₁-C₉ -chains or comprise cyclic alkyl groups, such as cycloalkyl-alkyl.

7. A pharmaceutical composition according to claim 6, wherein the acid susceptible proton pump inhibitor is selected from any one of

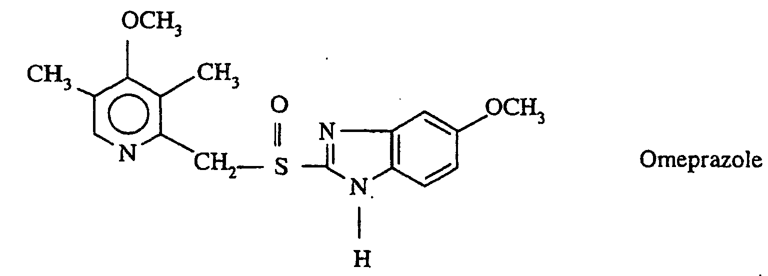

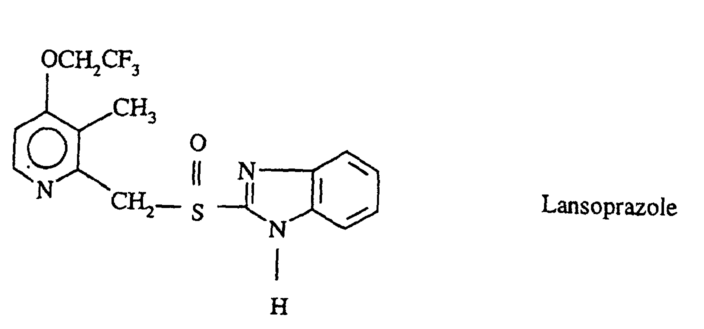

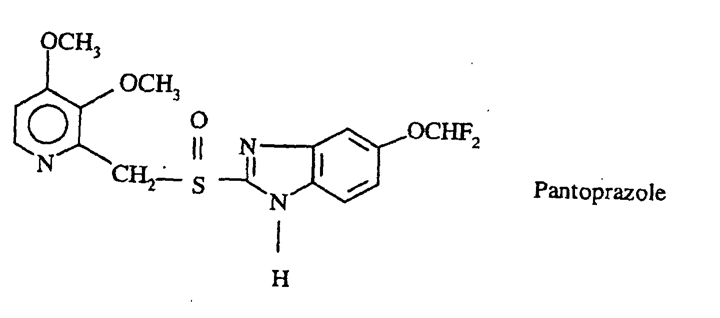

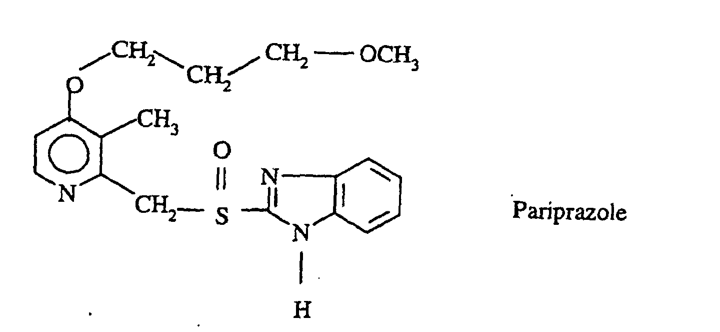

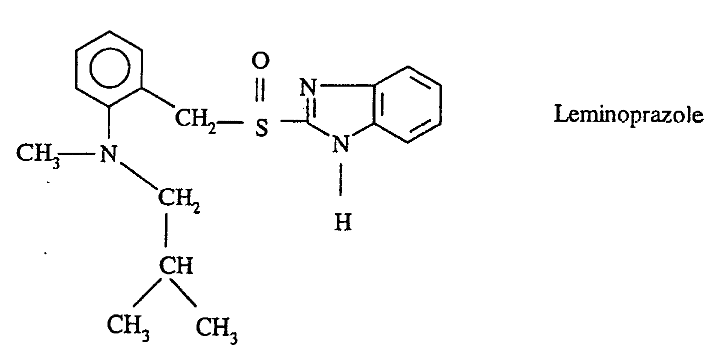

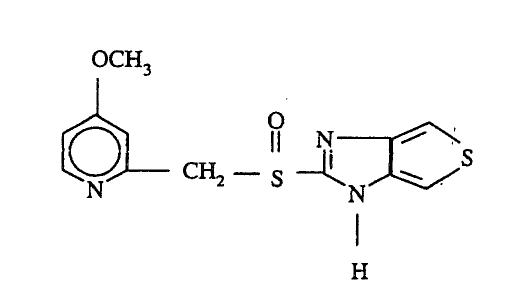

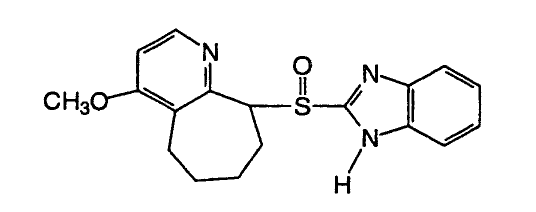

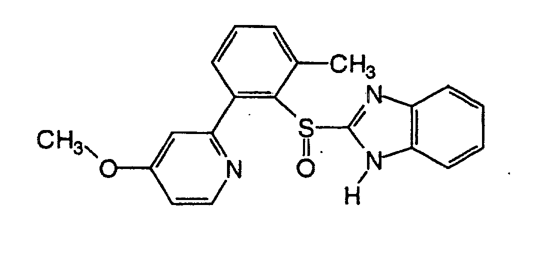

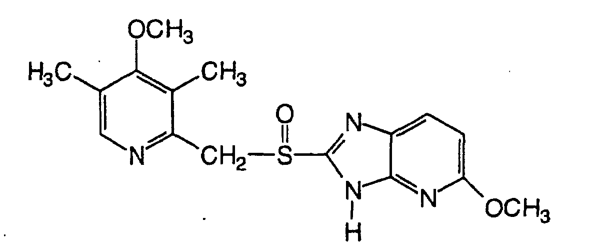

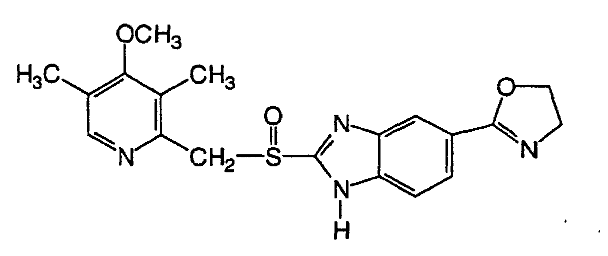

8. A pharmaceutical composition according to claim 7, wherein the acid susceptible proton pump inhibitor is selected from omeprazole, an alkaline salt of omeprazole, (S)-omeprazole and an alkaline salt of (S)-omeprazole.

9. A pharmaceutical composition according to claim 8, wherein the alkaline salt of omeprazole or (S)-omeprazole is a magnesium salt.

10. A pharmaceutical composition according to claim 5, wherein the NO-releasing NSAID is a compound of formula Ia and the acid susceptible proton pump inhibitor is selected from omeprazole, an alkaline salt of omeprazole, (S)-omeprazole and an alkaline salt of (S)-omeprazole.

11. A pharmaceutical compositon according to any one of the preceding claims, wherein the amount of the NO-releasing NSAID is from 50-1500 mg per unit dose.

12. A pharmaceutical compositon according to claim 11, wherein the amount of the NO-releasing NSAID is from 125-500 mg per unit dose.

13. A pharmaceutical compositon according to any one of the preceding claims, wherein the surfactant is a block co-polymer.

14. A pharmaceutical compositon according to any one of the preceding claims, wherein the surfactant is a non-ionic surfactant.

15. A pharmaceutical composition according to claim 14, wherein the non-ionic surfactant is a poloxamer.

16. A pharmaceutical compositon according to claim 14, wherein the surfactant is selected from any one of Poloxamer 407; Poloxamer 401; Poloxamer 237; Poloxamer 338; Poloxamer 331; Poloxamer 231; Poloxamine 908; Poloxamine 1307; Poloxamine 1107; and polyoxyethylene polyoxybutylene block copolymer.

17. A pharmaceutical compositon according to any one of the preceding claims, wherein the total amount of surfactant(s) is from 12.5-6000 mg.

18. A pharmaceutical compositon according to claim 17, wherein the total amount of surfactant(s) is from 100-500 mg.

19. A pharmaceutical compositon according to any one of the preceding claims, wherein the ratio NO-releasing NSAID: surfactant is within the range of from 1:0.1 - 1:10.

20. A pharmaceutical compositon according to claim 19 wherein the ratio NO-releasing NSAID: surfactant is within the range of from 1:0.3 -1:3.

21. A pharmaceutical compositon according to any one of the preceding claims, wherein an oil is present.

22. A pharmaceutical compositon according to claim 21, wherein the oil is a vegetable oil.

23. A pharmaceutical compositon according to claim 22, wherein the vegetable oil is selected from coconut oil, corn oil, soybean oil, rape seed oil, safflower oil and castor oil.

24. A pharmaceutical composition according to claim 21, wherein the oil is an animalic oil.

25. A pharmaceutical composition according to claim 24, wherein the animalic oil is a fish oil or one or more mono-, di- or triglycerides.

26. A pharmaceutical composition according to any one of the preceding claims, wherein a semi-solid fat is used as filler.

27. A pharmaceutical composition according to claim 26, wherein the semi-solid fat is selected from mono-, di- and triglycerides.

28. A pharmaceutical composition according to claim 27, wherein the mono-, di- and triglycerides are selected from glyceryl palmitostearate, or a mixture of mono-, di and triesters of glycerol, mono- and di-esters of polyethylene glycol or free polyethylene glycol.

29. A pharmaceutical composition according to any one of claims 2-28, wherein the short-chain alcohol is selected from ethanol, propyleneglycol or glycerol.

30. A pharmaceutical composition according to any one of the preceding claims, further comprising a co-surfactant.

31. A unit dosage form filled with a pharmaceutical composition according to any one of the preceding claims.

32. A unit dosage form according to claim 31, selected from any one of capsules, drinking ampoules, dose cushion, chewable soft pill, and chewy-base lozenges.

33. A unit dosage form according to claim 32, in form of a capsule.

34. A unit dosage form according to claim 33, wherein said capsule is a hard gelatine capsule.

35. A unit dosage form according to claim 33, wherein said capsule is a soft gelatine capsule.

36. A kit comprising a pharmaceutical composition according to claim 1 in a unit dosage form, in combination with an acid susceptible proton pump inhibitor.

37. A kit according to claim 36, wherein the proton pump inhibitor is enteric coated.

38. A kit according to claim 37, wherein the proton pump inhibitor is enteric coated omeprazol.

39. Use of a pharmaceutical composition according to any one of the preceding claims for the manufacturing of a medicament for the treatment of pain.

40. Use of a pharmaceutical composition according to any one of the preceding claims for the manufacturing of a medicament for the treatment of inflammation.

## Patentansprüche

1. Für die orale Verabreichung geeignete pharmazeutische Zusammensetzung in Form eines Emulsionsvorkonzentrats, enthaltend
(i) ein oder mehrere NO freisetzende (N) nichtsteroidale antiinflammatorisch wirkende Medikament(e) [NSAID(s)], wobei es sich bei dem NO freisetzenden NSAID um eine Verbindung der Formel I 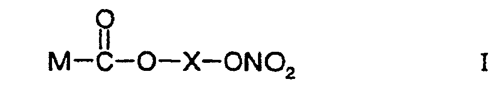 handelt, wobei
X für einen Spacer steht und
M aus den folgenden Resten ausgewählt ist 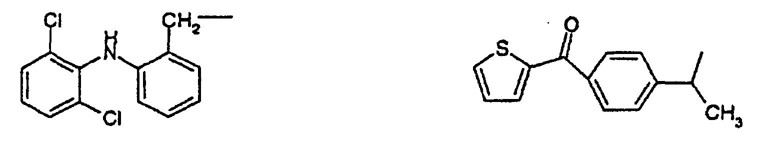 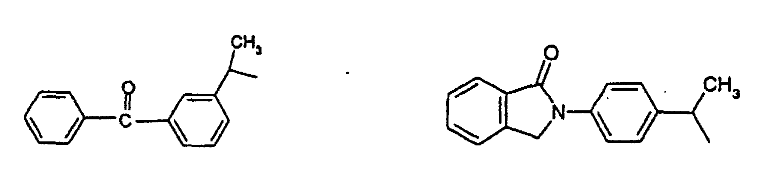 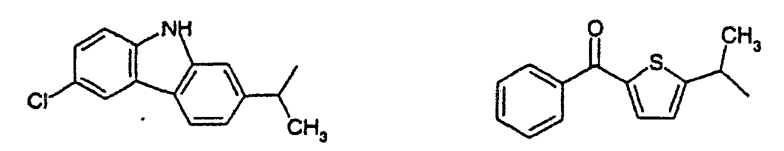 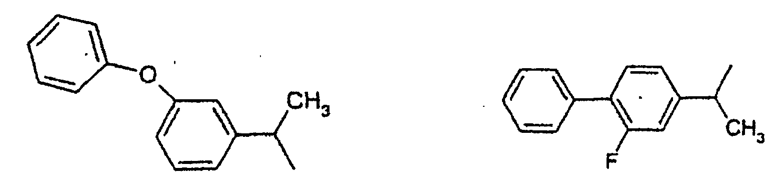 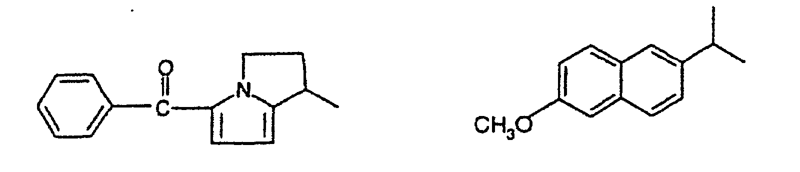 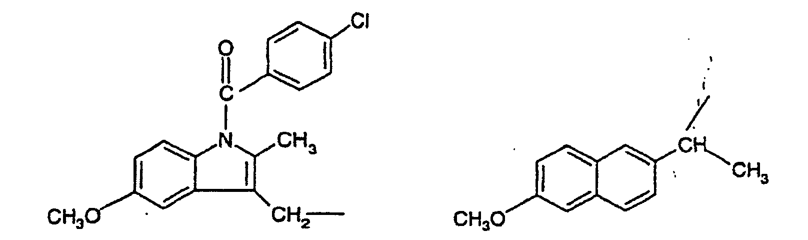 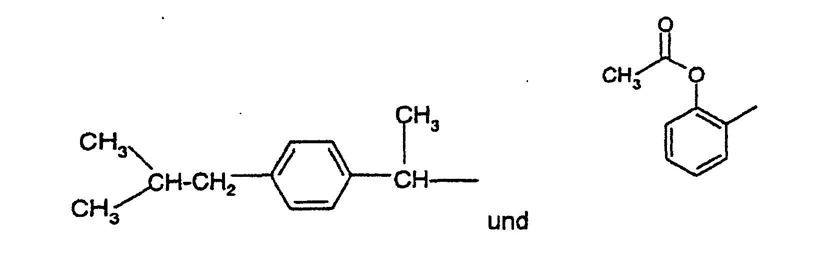
(ii) ein oder mehrere Tenside;
(iii)gegebenenfalls ein Öl oder ein halbfestes Fett;
wobei die Zusammensetzung, wenn sie mit wäßrigen Medien wie gastrointestinalen Flüssigkeiten in Kontakt kommt, in situ eine Öl-in-Wasser-Emulsion bildet.

2. Pharmazeutische Zusammensetzung nach Anspruch 1, weiterhin enthaltend einen oder mehrere kurzkettige Alkohole.

3. Pharmazeutische Zusammensetzung nach Anspruch 1 oder 2, wobei der Spacer X des NO freisetzenden NSAID aus geradkettigen, verzweigten oder cyclischen Alkengruppen -(CH₂)ₙ, wobei n für eine ganze Zahl von 2 bis 10 steht; -(CH₂)ₘ-O-(CH₂)ₚ-, wobei m und p für ganze Zahlen von 2 bis 10 stehen; und -CH₂-pC₆H₄-CH₂- ausgewählt ist.

4. Pharmazeutische Zusammensetzung nach einem der vorhergehenden Ansprüche, wobei es sich bei dem NO freisetzenden NSAID um eine aus den folgenden Verbindungen ausgewählte Verbindung handelt 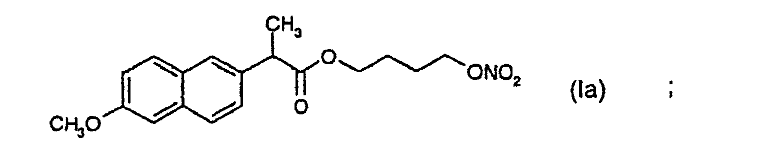 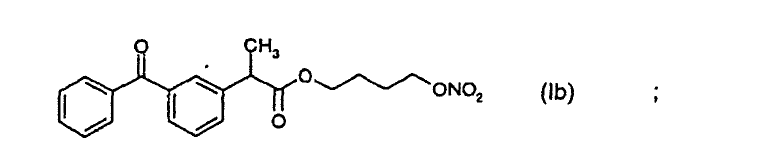 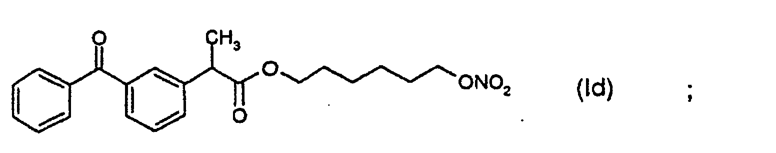 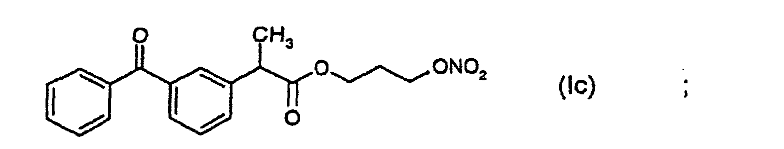 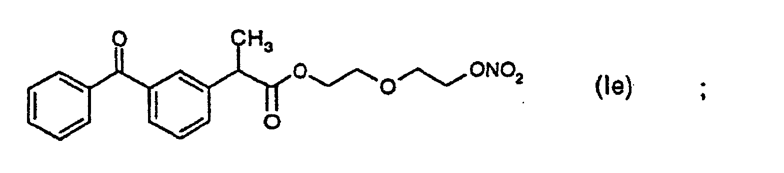 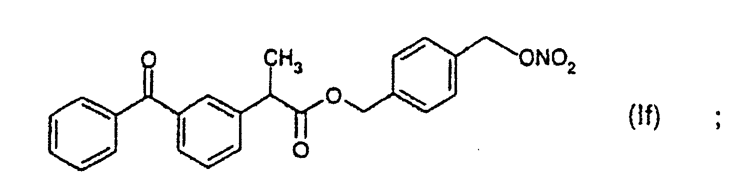 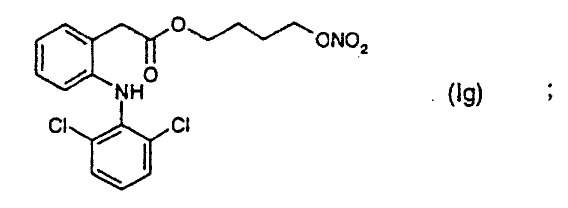 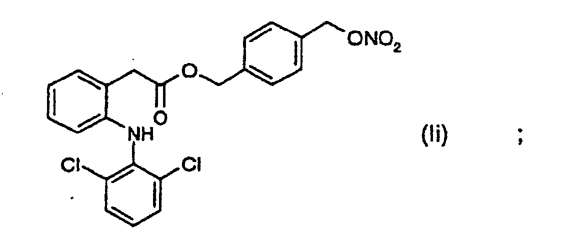 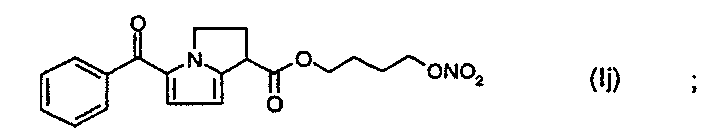 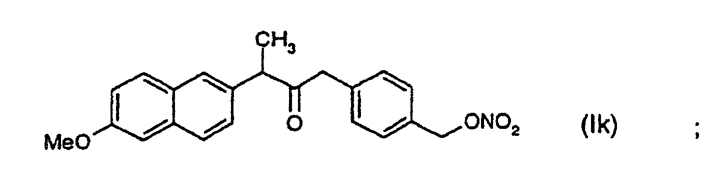 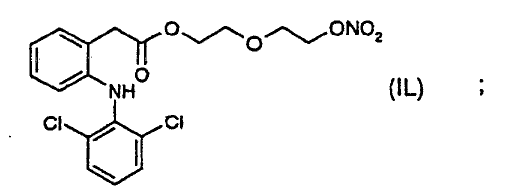 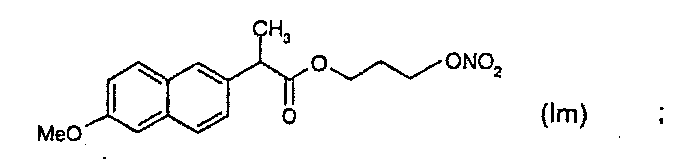 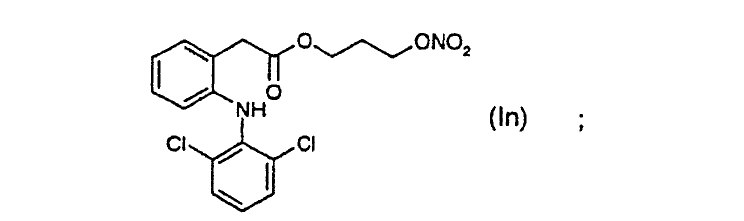 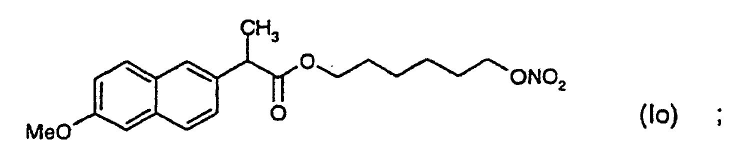 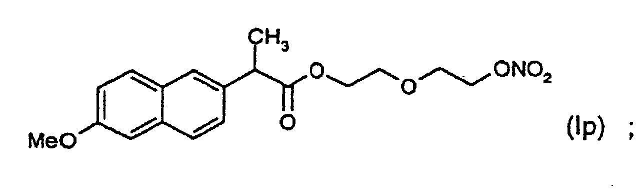 und

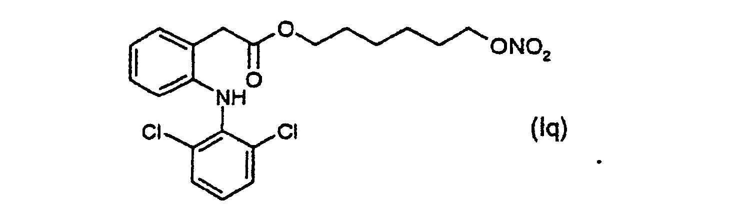

5. Pharmazeutische Zusammensetzung nach einem der vorhergehenden Ansprüche, weiterhin enthaltend individuell magensaftresistent beschichtete Einheiten eines säureempfindlichen Protonpumpenhemmers oder eines pharmazeutisch unbedenklichen alkalischen Salzes davon.

6. Pharmazeutische Zusammensetzung nach Anspruch 5, wobei der säureempfindliche Protonpumpenhemmer aus Verbindungen der allgemeinen Formel I und deren pharmazeutisch unbedenklichen alkalischen Salzen und einzelnen Enantiomeren davon und alkalischen Salzen der einzelnen Enantiomeren ausgewählt ist 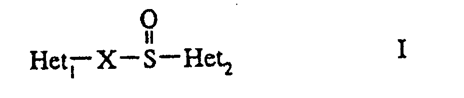 wobei
Het₁ für 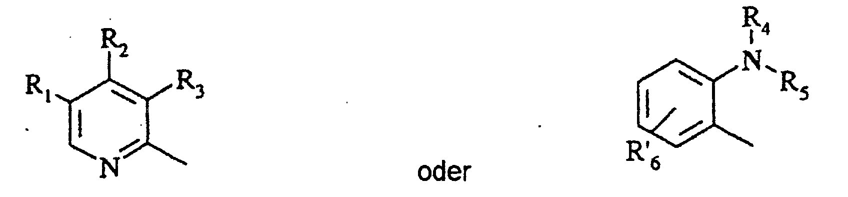 steht;
Het₂ für 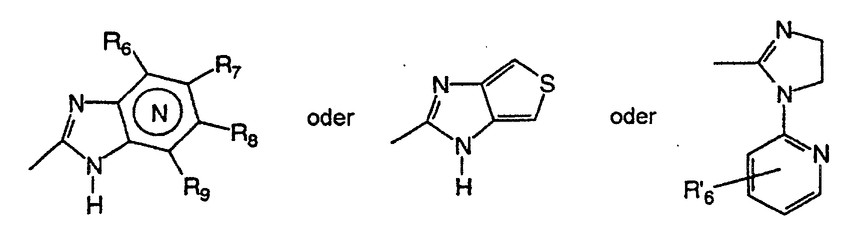 steht;
X = 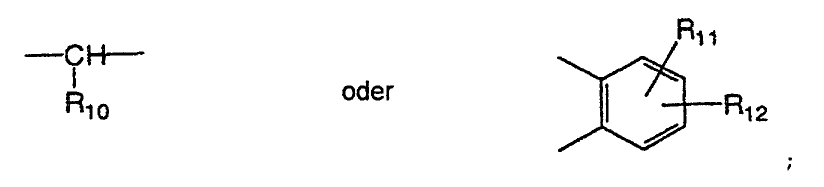
wobei
N in der Benzimidazolgruppe bedeutet, daß eines der durch R₆-R₉ substituierten Kohlenstoffatome gegebenenfalls gegen ein Stickstoffatom ohne Substituenten ausgetauscht werden kann;
R₁, R₂ und R₃ gleich oder verschieden sind und aus Wasserstoff, Alkyl, Alkoxy, gegebenenfalls substituiert durch Fluor, Alkylthio, Alkoxyalkoxy, Dialkylamino, Piperidino, Morpholino, Halogen, Phenyl und Phenylalkoxy ausgewählt sind;
R₄ und R₅ gleich oder verschieden sind und aus Wasserstoff, Alkyl und Aralkyl ausgebildet sind;
R₆' für Wasserstoff, Halogen, Trifluormethyl, Alkyl und Alkoxy steht;
R₆-R₉ gleich oder verschieden sind und aus Wasserstoff, Alkyl, Alkoxy, Halogen, Halogenalkoxy, Alkylcarbonyl, Alkoxycarbonyl, Oxazolyl, Trifluoralkyl ausgewählt sind oder benachbarte Gruppen R₆-R₉ Ringstrukturen bilden, die weiter substituiert sein können;
R₁₀ für Wasserstoff steht oder zusammen mit R₃ eine Alkylenkette bildet und
R₁₁ und R₁₂ gleich oder verschieden sind und aus Wasserstoff, Halogen und Alkyl ausgewählt sind;
wobei Alkylgruppen, Alkoxygruppen und Teile davon verzweigte oder geradkettige C₁-C₉-Ketten sein oder cyclische Alkylgruppen enthalten können, wie z.B. Cycloalkylalkyl.

7. Pharmazeutische Zusammensetzung nach Anspruch 6, wobei der säureempfindliche Protonpumpenhemmer aus den folgenden Verbindungen ausgewählt ist:

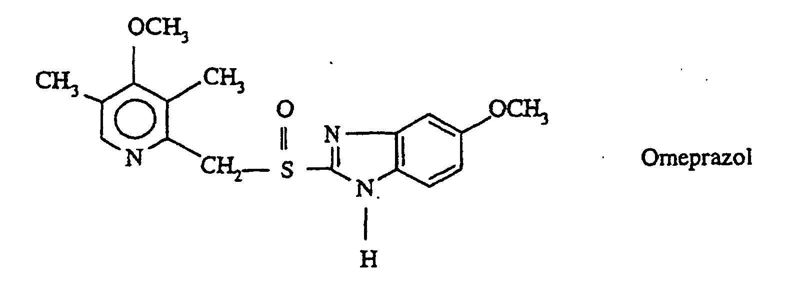

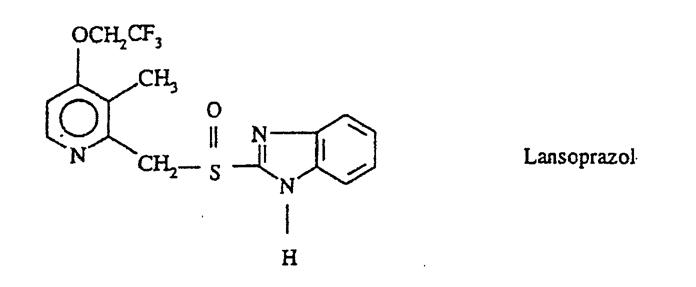

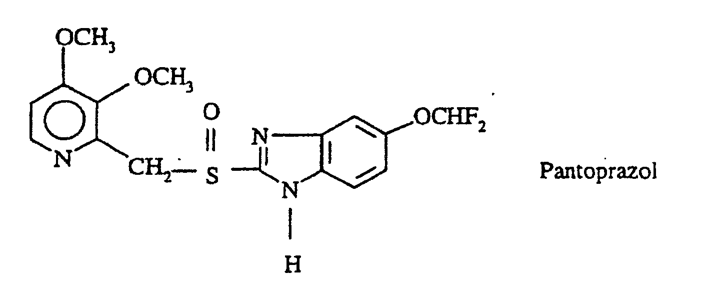

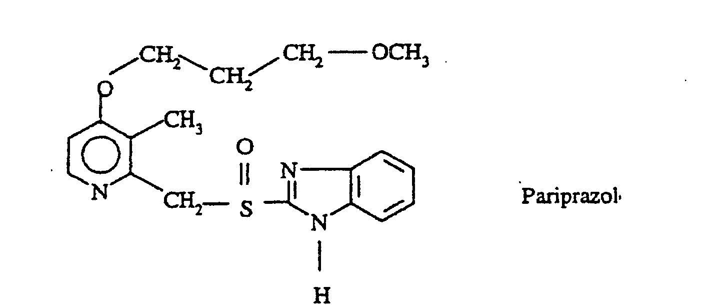

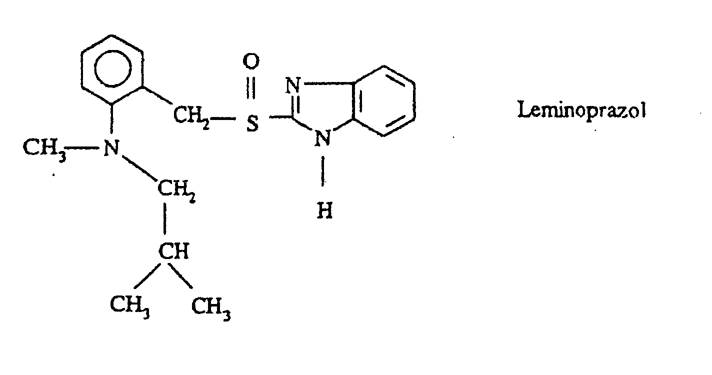

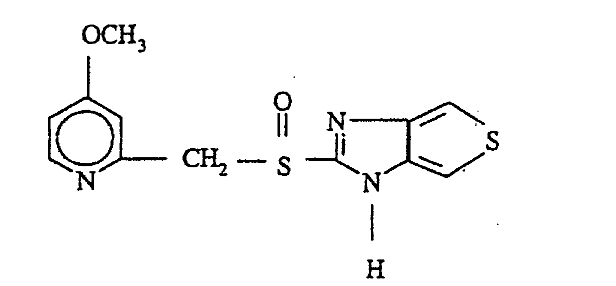

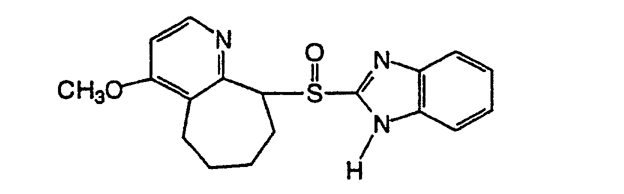

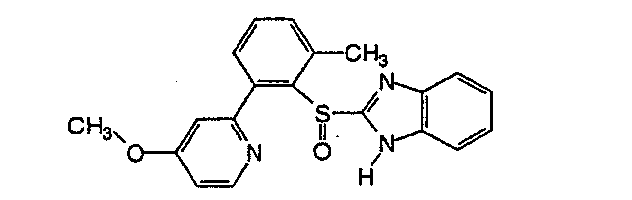

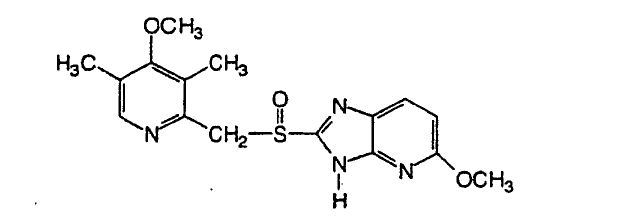

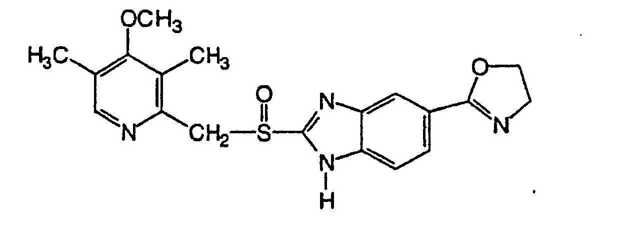

8. Pharmazeutische Zusammensetzung nach Anspruch 7, wobei der säureempfindliche Protonenpumpenhemmer aus Omeprazol, einem alkalischen Salz von Omeprazol, (S)-Omeprazol und einem alkalischen Salz von (S)-Omeprazol ausgebildet ist.

9. Pharmazeutische Zusammensetzung nach Anspruch 8, wobei es sich bei dem alkalischen Salz von dem Omeprazol oder (S)-Omeprazol um ein Magnesiumsalz handelt.

10. Pharmazeutische Zusammensetzung nach Anspruch 5, wobei es sich bei dem NO freisetzenden NSAID um eine Verbindung der Formel Ia handelt und der säureempfindliche Protonenpumpenhemmer aus Omeprazol, einem alkalischen Salz von dem Omeprazol, (S)-Omeprazol und einem alkalischen Salz von (S)-Omeprazol ausgewählt ist.

11. Pharmazeutische Zusammensetzung nach einem der vorhergehenden Ansprüche, wobei die Menge an NO freisetzendem NSAID 50-1500 mg pro Einheitsdosis beträgt.

12. Pharmazeutische Zusammensetzung nach Anspruch 11, wobei die Menge an NO freisetzendem NSAID 125-500 mg pro Einheitsdosis beträgt.

13. Pharmazeutische Zusammensetzung nach einem der vorhergehenden Ansprüche, wobei es sich bei dem Tensid um ein Blockcopolymer handelt.

14. Pharmazeutische Zusammensetzung nach einem der vorhergehenden Ansprüche, wobei es sich bei dem Tensid um ein nichtionisches Tensid handelt.

15. Pharmazeutische Zusammensetzung nach Anspruch 14, wobei es sich bei dem nichtionischen Tensid um ein Poloxamer handelt.

16. Pharmazeutische Zusammensetzung nach Anspruch 14, wobei das Tensid aus einer der folgenden Verbindungen ausgewählt ist: Poloxamer 407; Poloxamer 401; Poloxamer 237; Poloxamer 338; Poloxamer 331; Poloxamer 231; Poloxamin 908; Poloxamin 1307; Poloxamin 1107; und Polyoxyethylen-Polyoxybutylen-Blockcopolymer.

17. Pharmazeutische Zusammensetzung nach einem der vorhergehenden Ansprüche, wobei die Gesamtmenge an Tensid(en) 12,5-6000 mg beträgt.

18. Pharmazeutische Zusammensetzung nach Anspruch 17, wobei die Gesamtmenge an Tensid(en) 100-500 mg beträgt.

19. Pharmazeutische Zusammensetzung nach einem der vorhergehenden Ansprüche, wobei das Verhältnis von NO freisetzendem NSAID:Tensid im Bereich von 1:0,1 - 1:10 liegt.

20. Pharmazeutische Zusammensetzung nach Anspruch 19, wobei das Verhältnis von NO freisetzendem NSAID:Tensid im Bereich von 1:0,3 - 1:3 liegt.

21. Pharmazeutische Zusammensetzung nach einem der vorhergehenden Ansprüche, in der ein Öl vorliegt.

22. Pharmazeutische Zusammensetzung nach Anspruch 21, wobei es sich bei dem Öl um ein Pflanzenöl handelt.

23. Pharmazeutische Zusammensetzung nach Anspruch 22, wobei das Pflanzenöl aus Kokusnußöl, Maisöl, Sojabohnenöl, Rapsöl, Safloröl und Rizinusöl ausgewählt ist.

24. Pharmazeutische Zusammensetzung nach Anspruch 21, wobei es sich bei dem Öl um ein Tieröl handelt.

25. Pharmazeutische Zusammensetzung nach Anspruch 24, wobei es sich bei dem Tieröl um ein Fischöl oder ein oder mehrere Mono-, Di- oder Triglyceride handelt.

26. Pharmazeutische Zusammensetzung nach einem der vorhergehenden Ansprüche, wobei ein halbfestes Fett als Füllstoff verwendet wird.

27. Pharmazeutische Zusammensetzung nach Anspruch 26, wobei das halbfeste Fett aus Mono-, Di- und Triglyceriden ausgebildet ist.

28. Pharmazeutische Zusammensetzung nach Anspruch 27, wobei die Mono-, Di- und Triglyceride aus Glycerylpalmitostearat und einer Mischung von Mono-, Di- und Triestern von Glycerin, Mono- und Diestern von Polyethylenglycol oder freiem Polyethylenglycol ausgewählt sind.

29. Pharmazeutische Zusammensetzung nach einem der Ansprüche 2-28, wobei der kurzkettige Alkohol aus Ethanol, Propylenglycol und Glycerol ausgewählt ist.

30. Pharmazeutische Zusammensetzung nach einem der vorhergehenden Ansprüche, weiterhin enthaltend ein Cotensid.

31. Einheitsdosisform, gefüllt mit einer pharmazeutischen Zusammensetzung nach einem der vorhergehenden Ansprüche.

32. Einheitsdosisform nach Anspruch 31, ausgewählt aus Kapseln, Trinkampullen, Dosiskissen, weichen Kaupillen und Lutschtabletten mit kaubarer Basis.

33. Einheitsdosisform nach Anspruch 32 in Form einer Kapsel.

34. Einheitsdosisform nach Anspruch 33, wobei es sich bei der Kapsel um eine Hartgelatinekapsel handelt.

35. Einheitsdosisform nach Anspruch 33, wobei es sich bei der Kapsel um eine Weichgelatinekapsel handelt.

36. Kit, enthaltend eine pharmazeutische Zusammensetzung nach Anspruch 1 in einer Einheitsdosisform in Kombination mit einem säureempfindlichen Protonpumpenhemmer.

37. Kit nach Anspruch 36, wobei der Protonpumpenhemmer magensaftresistent beschichtet ist.

38. Kit nach Anspruch 37, wobei es sich bei dem Protonpumpenhemmer um magensaftresistent beschichtetes Omeprazol handelt.

39. Verwendung einer pharmazeutischen Zusammensetzung nach einem der vorhergehenden Ansprüche zur Herstellung eines Medikaments zur Behandlung von Schmerzen.

40. Verwendung einer pharmazeutischen Zusammensetzung nach einem der vorhergehenden Ansprüche zur Herstellung eines Medikaments zur Behandlung von Entzündungen.

## Revendications

1. Composition pharmaceutique convenable pour une administration orale, sous la forme d'un pré-concentré d'émulsion, comprenant.
(i) un ou plusieurs AINS à libération de NO, dans lesquels le AINS à libération de NO est un composé de formule I 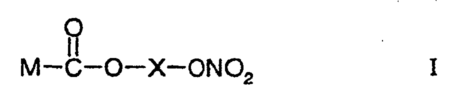 dans laquelle
X est un espaceur ; et
M est choisi parmi un quelconque parmi 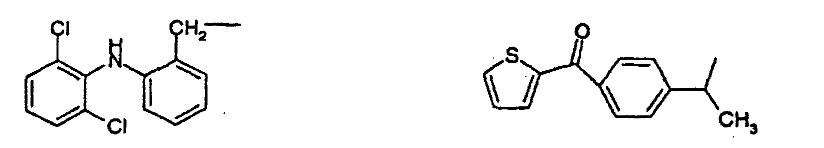 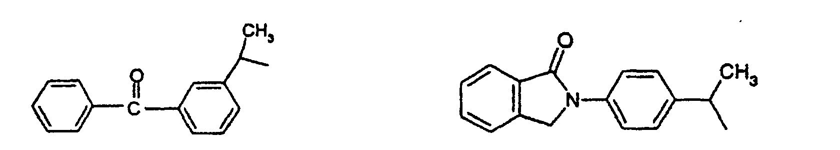 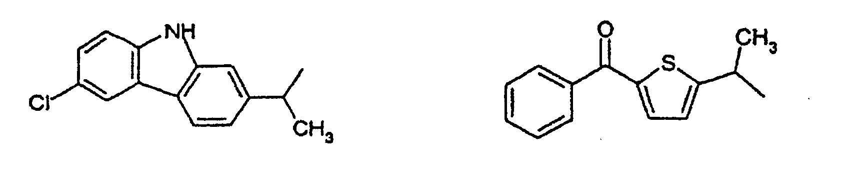 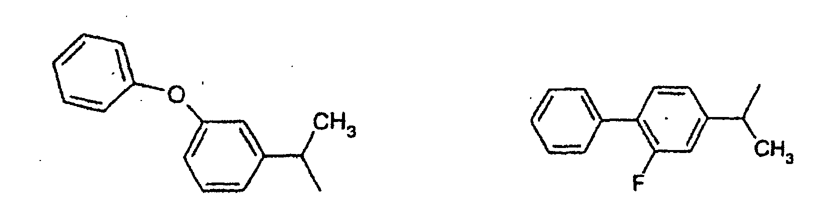 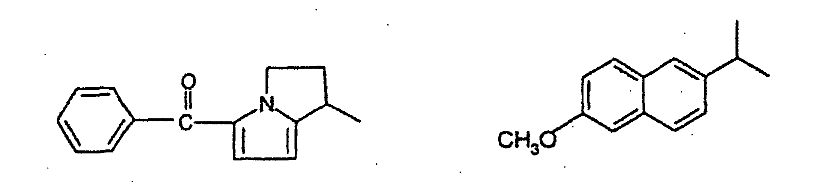 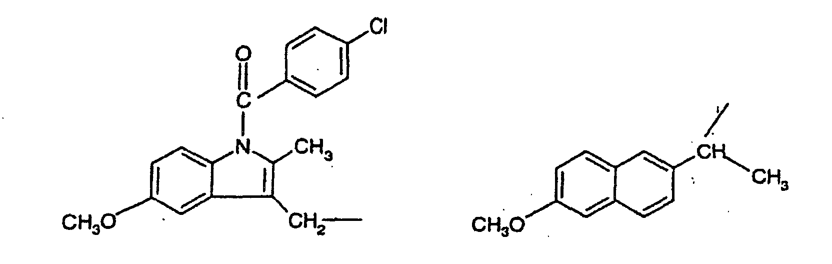 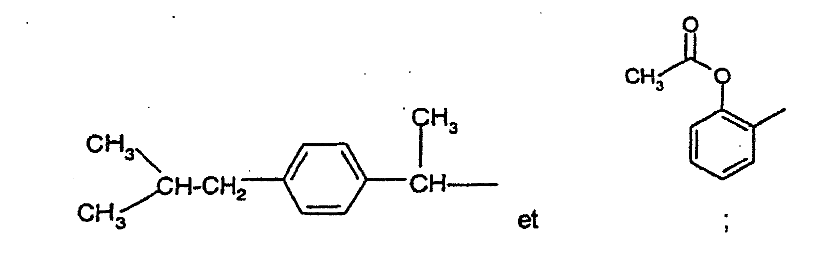
(ii) un ou plusieurs agents tensioactifs ;
(iii) éventuellement une huile ou une matière grasse semi-solide ;
ladite composition formant in situ une émulsion huile-dans-eau lors d'un contact avec des milieux aqueux tels que les liquides gastro-intestinaux.

2. Composition pharmaceutique selon la revendication 1, comprenant en outre un ou plusieurs alcools à chaîne courte.

3. Composition pharmaceutique selon la revendication 1 ou 2, dans laquelle l'espaceur X du AINS à libération de NO est choisi parmi un groupement alkylène -(CH₂)-ₙ linéaire, ramifié ou cyclique, dans lequel n est un nombre entier allant de 2 à 10 ; -(CH₂)ₘ-O-(CH2)ₚ- dans lequel m et p sont des nombres entiers allant de 2 à 10 ; et -CH₂-pC₆H₄-CH₂- .

4. Composition pharmaceutique selon l'une quelconque des revendications précédentes, dans laquelle l'AINS à libération de NO est un composé quelconque choisi parmi 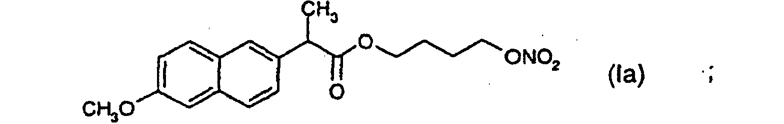 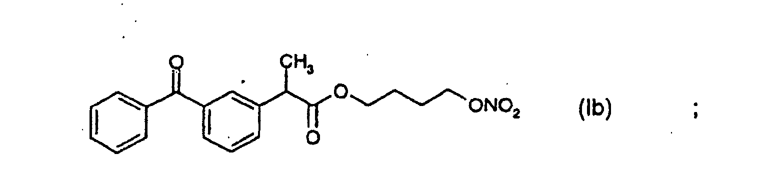 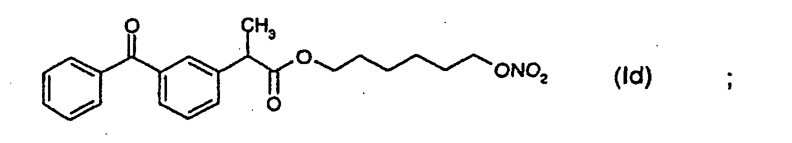 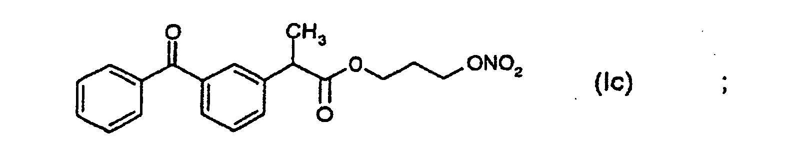 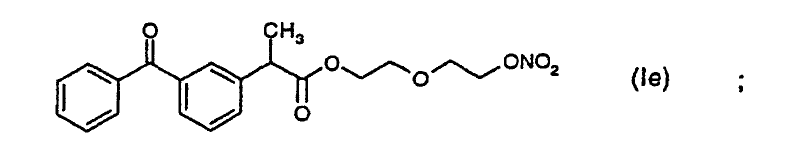 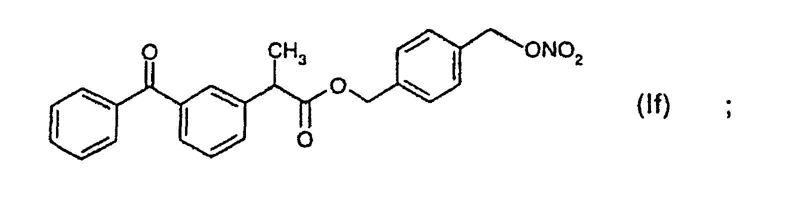 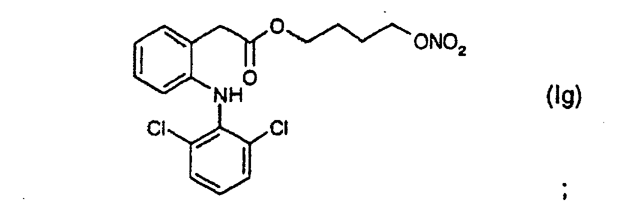 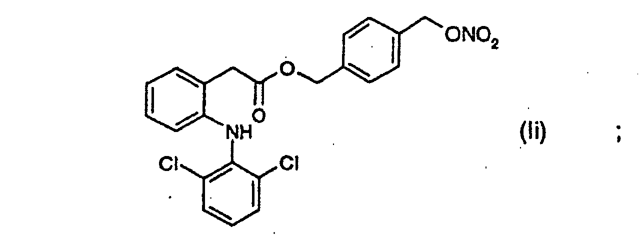 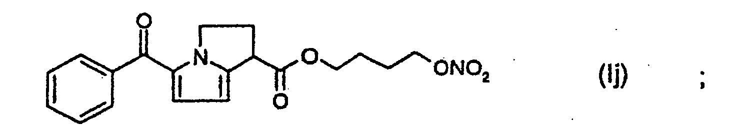 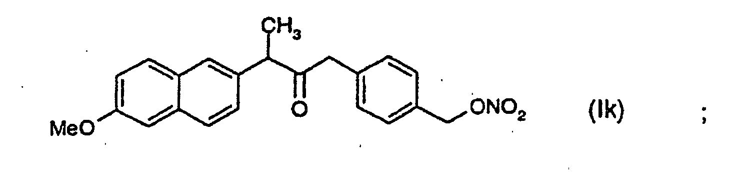 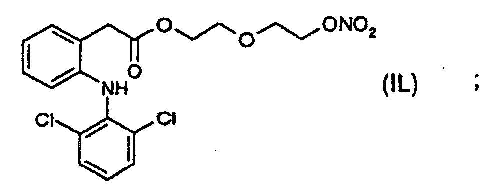 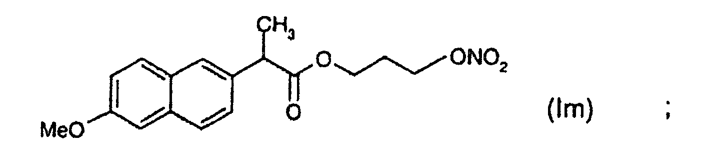 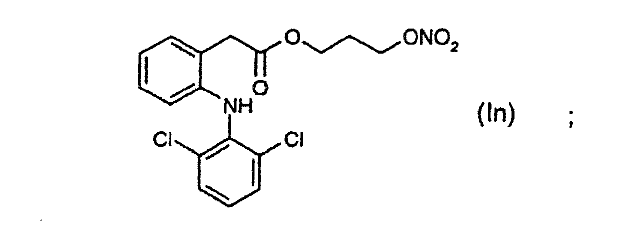 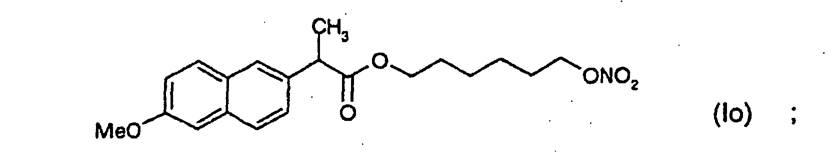 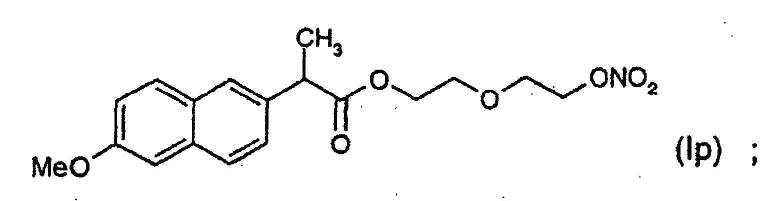 et

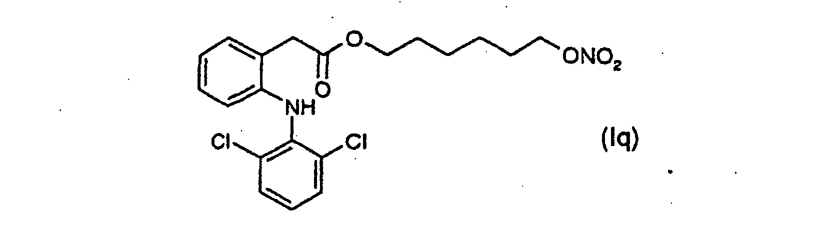

5. Composition pharmaceutique selon l'une quelconque des revendications précédentes, comprenant en outre des unités, individuellement recouvertes d'un enrobage entérique, d'un inhibiteur de pompe à protons sensible à l'acide, ou d'un sel alcalin pharmaceutiquement acceptable de celui-ci.

6. Composition pharmaceutique selon la revendication 5, dans laquelle l'inhibiteur de pompe à protons sensible à l'acide est choisi parmi un composé de formule générale I ou un sel alcalin pharmaceutiquement acceptable de celui-ci, ou l'un de ses énantiomères uniques ou un sel alcalin de l'énantiomère unique. 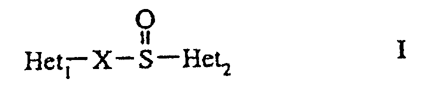 dans laquelle
Hét₁ est 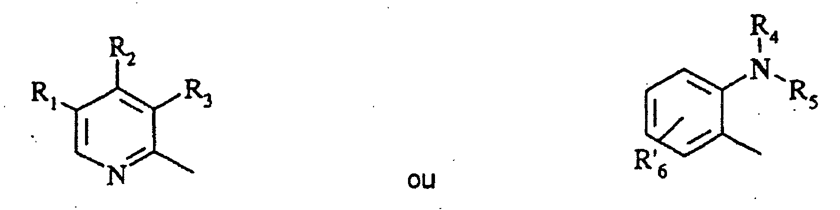
Hét₂ est 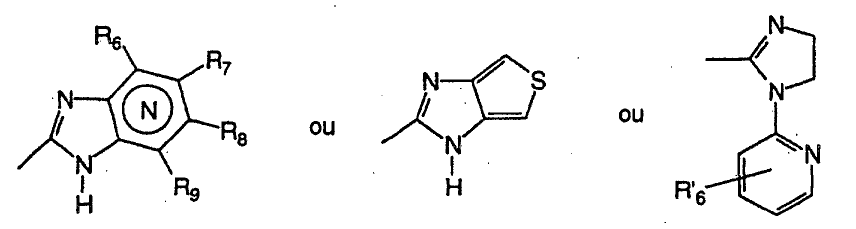
X est 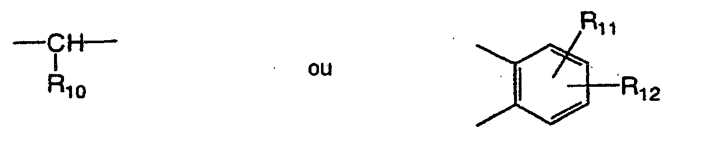
dans lesquelles
N dans le motif benzimidazole signifie que l'un des atomes de carbone substitué par R₆-R₉ peut éventuellement être échangé pour un atome d'azote sans aucun substituant ;
R₁, R₂ et R₃ sont identiques ou différents et sont choisis parmi hydrogène, alkyle, alcoxy éventuellement substitué par fluor, alkylthio, alcoxyalcoxy, dialkylamino, pipéridino, morpholino, halogène, phényle et phénylalcoxy ;
R₄ et R₅ sont identiques ou différents et sont choisis parmi hydrogène, alkyle et aralkyle ;
R₆' est hydrogène, halogène, trifluorométhyle, alkyle et alcoxy ;
R₆-R₉ sont identiques ou différents et sont choisis parmi hydrogène, alkyle, alcoxy, halogène, halogénoalcoxy, alkylcarbonyle, alcoxycarbonyle, oxazolyle, trifluoroalkyle, ou les groupements adjacents R₆-R₉ forment des structures cycliques pouvant être substituées davantage ;
R₁₀ est hydrogène ou forme une chaîne alkylène conjointement avec R₃, et
R₁₁ et R₁₂ sont identiques ou différents et sont choisis parmi hydrogène, halogène ou alkyle ; des groupements alkyle, des groupements alcoxy et des motifs de ceux-ci, ils peuvent être des chaînes en C₁-C₉ linéaires ou ramifiées ou comprendre des groupements alkyle cycliques, tels que cycloalkylalkyle.

7. Composition pharmaceutique selon la revendication 6, dans laquelle l'inhibiteur de pompe à protons sensible à l'acide est choisi parmi l'un quelconque parmi

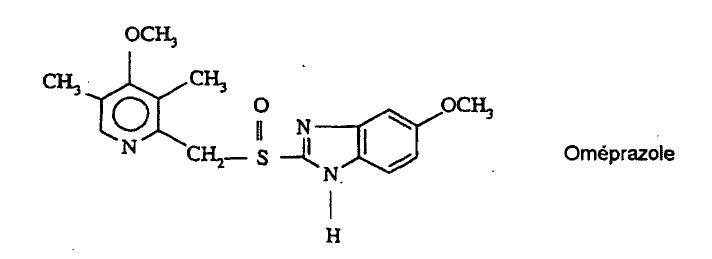

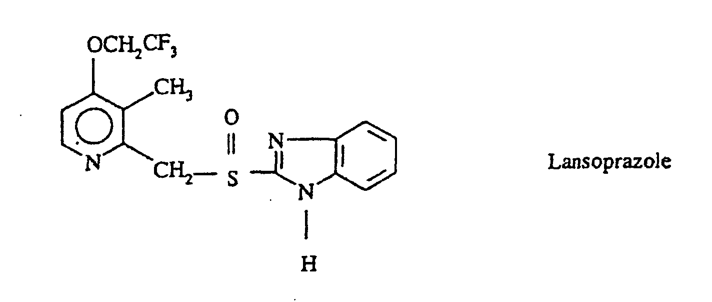

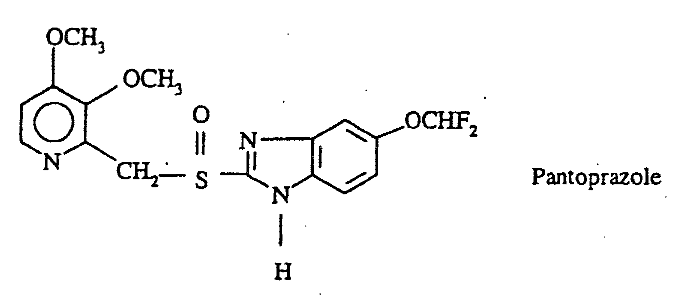

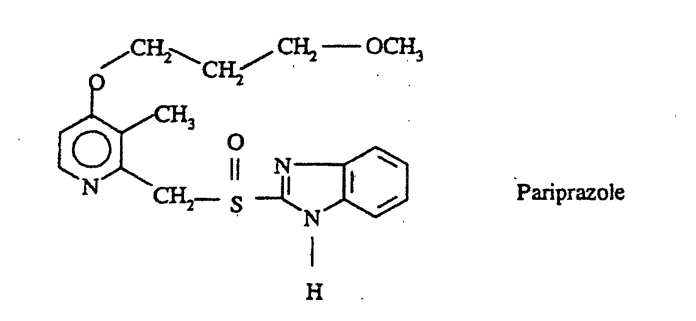

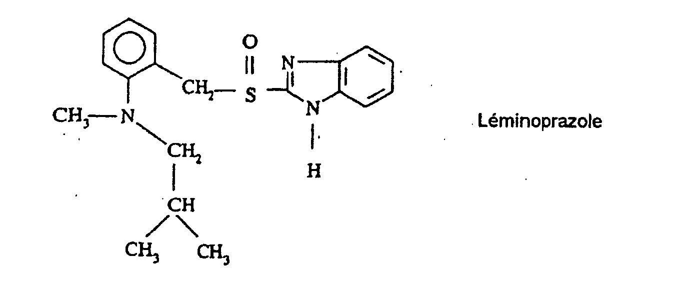

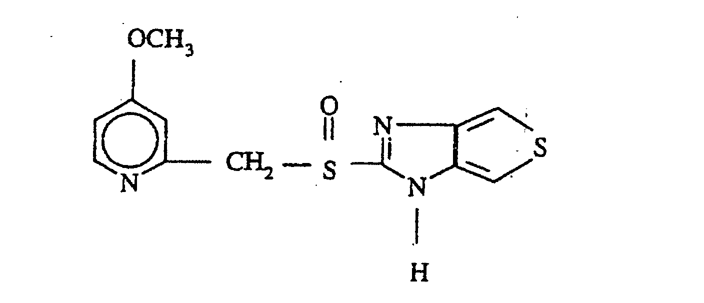

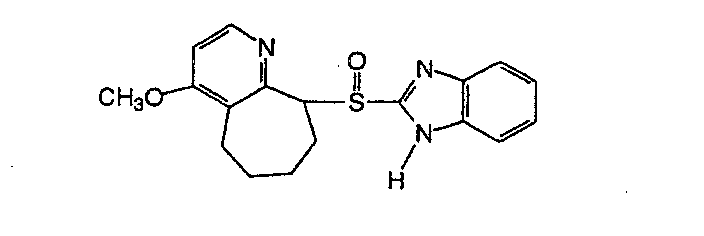

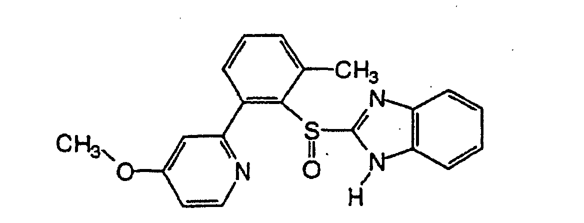

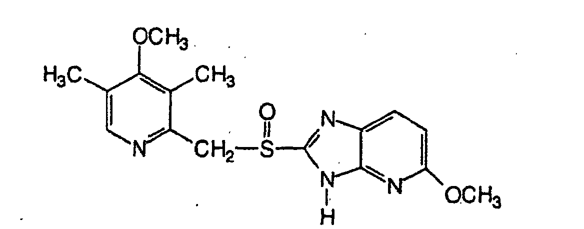

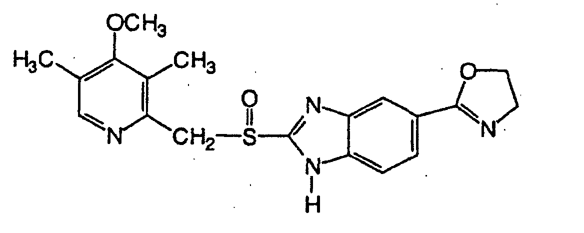

8. Composition pharmaceutique selon la revendication 7, dans laquelle l'inhibiteur de pompe à protons sensible à l'acide est choisi parmi l'oméprazole, un sel alcalin de l'oméprazole, le (S)-oméprazole et un sel alcalin du (S)-oméprazole.

9. Composition pharmaceutique selon la revendication 8, dans laquelle le sel alcalin de l'oméprazole ou du (S)-oméprazole est un sel de magnésium.

10. Composition pharmaceutique selon la revendication 5, dans laquelle l'AINS à libération de NO est un composé de formule Ia et l'inhibiteur de pompe à protons sensible à l'acide est choisi parmi l'oméprazole, un sel alcalin de l'oméprazole, le (S)-oméprazole et un sel alcalin du (S)-oméprazole.

11. Composition pharmaceutique selon l'une quelconque des revendications précédentes, dans laquelle la quantité d'AINS à libération de NO va de 50 à 1500 mg par dose unitaire.

12. Composition pharmaceutique selon la revendication 11, dans laquelle la quantité d'AINS à libération de NO va de 125 à 500 mg par dose unitaire.

13. Composition pharmaceutique selon l'une quelconque des revendications précédentes, dans laquelle l'agent tensioactif est un copolymère bloc.

14. Composition pharmaceutique selon l'une quelconque des revendications précédentes, dans laquelle l'agent tensioactif est un agent tensioactif non ionique.

15. Composition pharmaceutique selon la revendication 14, dans laquelle l'agent tensioactif non ionique est un poloxamère.

16. Composition pharmaceutique selon la revendication 14, dans laquelle l'agent tensioactif est choisi parmi un quelconque parmi le Poloxamère 407 ; le Poloxamère 401 ; le Poloxamère 237 ; le Poloxamère 338 ; le Poloxamère 331 ; le Poloxamère 231 ; la Poloxamine 908 ; la Poloxamine 1307 ; la Poloxamine 1107 ; et le copolymère bloc de polyoxyéthylène et de polyoxybutylène.

17. Composition pharmaceutique selon l'une quelconque des revendications précédentes, dans laquelle la quantité totale d'agent(s) tensioactif(s) va de 12,5 à 6000 mg.

18. Composition pharmaceutique selon la revendication 17, dans laquelle la quantité totale d'agent(s) tensioactif(s) va de 100 à 500 mg.

19. Composition pharmaceutique selon l'une quelconque des revendications précédentes, dans laquelle le rapport de l'AINS à libération de NO à l'agent tensioactif est au sein de la plage allant de 1:0,1 à 1:10.

20. Composition pharmaceutique selon la revendication 19, dans laquelle le rapport de l'AINS à libération de NO à l'agent tensioactif est au sein de la plage allant de 1:0,3 à 1:3.

21. Composition pharmaceutique selon l'une quelconque des revendications précédentes, dans laquelle une huile est présente.

22. Composition pharmaceutique selon la revendication 21, dans laquelle l'huile est une huile végétale.

23. Composition pharmaceutique selon la revendication 22, dans laquelle l'huile végétale est choisie parmi l'huile de coprah, l'huile de maïs, l'huile de soja, l'huile de colza, l'huile de carthame et l'huile de ricin.

24. Composition pharmaceutique selon la revendication 21, dans laquelle l'huile est une huile animale.

25. Composition pharmaceutique selon la revendication 24, dans laquelle l'huile animale est une huile de poisson ou un ou plusieurs mono-, di- ou triglycérides.

26. Composition pharmaceutique selon l'une quelconque des revendications précédentes, dans laquelle une matière grasse semi-solide est utilisée comme charge.

27. Composition pharmaceutique selon la revendication 26, dans laquelle la matière grasse semi-solide est choisie parmi les mono-, di- et triglycérides.

28. Composition pharmaceutique selon la revendication 27, dans laquelle les mono-, di- et triglycérides sont choisis parmi le palmitostéarate de glycéryle, ou un mélange de mono-, di- et triesters de glycérol, de mono- et diesters de polyéthylène glycol ou le polyéthylène glycol libre.

29. Composition pharmaceutique selon l'une quelconque des revendications 2 à 28, dans laquelle l'alcool à chaîne courte est choisi parmi l'éthanol, le propylène glycol ou le glycérol.

30. Composition pharmaceutique selon l'une quelconque des revendications précédentes, comprenant en outre un co-agent tensioactif.

31. Forme de dosage unitaire remplie d'une composition pharmaceutique selon l'une quelconque des revendications précédentes.

32. Forme de dosage unitaire selon la revendication 31, choisie parmi un ou une quelconque parmi des capsules, des ampoules buvables, un coussin de dosage, une pilule molle croquable, et des tablettes à base moelleuse.

33. Forme de dosage unitaire selon la revendication 32, sous la forme d'une capsule.

34. Forme de dosage unitaire selon la revendication 33, dans laquelle ladite capsule est une capsule de gélatine dure.

35. Forme de dosage unitaire selon la revendication 33, dans laquelle ladite capsule est une capsule de gélatine molle.

36. Kit comprenant une composition pharmaceutique selon la revendication 1 sous une forme de dosage unitaire, en association avec un inhibiteur de pompe à protons sensible à l'acide.

37. Kit selon la revendication 36, dans lequel l'inhibiteur de pompe à protons est à enrobage entérique.

38. Kit selon la revendication 37, dans lequel l'inhibiteur de pompe à protons est l'oméprazole à enrobage entérique.

39. Utilisation d'une composition pharmaceutique selon l'une quelconque des revendications précédentes, pour la fabrication d'un médicament pour le traitement de la douleur.

40. Utilisation d'une composition pharmaceutique selon l'une quelconque des revendications précédentes, pour la fabrication d'un médicament pour le traitement des inflammations.
